# EUROPEAN PATENT APPLICATION

(11) **EP 4 516 900 A1**
(43) Date of publication of application: **05.03.2025**
(21) Application number: 23795469.8
(22) Date of filing: 26.04.2023
(51) Int. Cl.: C12N 9/88, C12N 15/60, C12N 15/81, C12N 1/19, C12P 7/04, C12R 1/865

(54) **NEROLIDOL SYNTHASE AND USE THEREOF**

(30) Priority: 29.04.2022 CN 202210473488
(71) Applicant: Wuhan Hesheng Technology Co., Ltd., Wuhan, Hubei 430070 (CN)
(72) Inventor: YE, Ziling, Wuhan, Hubei 430070 (CN)
(74) Representative: Huang, Liwei
(86) International application number: PCT/CN2023/090827
(87) International publication number: WO 2023/208037

(57) **Abstract**

Provided are a nerolidol synthetase comprising structural domains having Pfam numbers PF01397 and PF03936, and a use thereof. The amino acid sequence of the nerolidol synthase is as shown in SEQ ID NO. 1, SEQ ID NO. 2, SEQ ID NO. 3, SEQ ID NO. 4, SEQ ID NO. 11 or SEQ ID NO. 12, and a nucleotide sequence encoding a nucleic acid molecule is as shown in SEQ ID NO. 5, SEQ ID NO. 6, SEQ ID NO. 7, SEQ ID NO. 8, SEQ ID NO. 9 or SEQ ID NO. 10. The use comprises: integrating a coding gene of the nerolidol synthetase into a nucleic acid construct and introducing same into a host cell to obtain a recombinant bacterium, so that the coding gene is expressed in the recombinant bacterium. Thus, biosynthesis of nerolidol is implemented, and the yield of nerolidol is remarkably improved.

## Description

### Field of the invention

The invention belongs to the field of nerolidol biosynthesis, and particularly relates to nerolidol synthase and use thereof.

### Background of the invention

Nerolidol is a sesquiterpene compound originally found in orange blossom, thus was named nerolidol, and was later found to exist in aromatic plants such as citronella, lavender, lemongrass and ginger. Nerolidol has a wide range of biological functions, such as acaricidal, anti-herbivorous, antibacterial, antioxidant, anti-inflammatory and anti-anxiety activities, and is a promising phytochemical drug. It is also a synthetic intermediate of herbivore-induced volatile substance DMNT, that can protect plants from damage of herbivores. In addition, nerolidol was approved by the U.S. Food and Drug Administration (FDA) as a food flavoring agent.

The synthesis of nerolidol includes three main methods: plant extraction, chemical synthesis and biosynthesis. Plant extraction and chemical synthesis are subjected to various limitations such as complex process, low yield due to seasonal effects, high extraction cost; in addition, plant extraction and chemical synthesis often involve toxic reagents in reactions, which have a great impact on the environment, and possibly higher amount of solvent residues and toxic reagent residues in the products. Thus, the safety of the product needs to be improved. Metabolic engineering and synthetic biology provide an alternative pathway for engineering microbial cell factories to produce nerolidol sustainably and efficiently.

### Summary of the invention

The aim of the present invention is to provide a nerolidol synthase and use thereof in the biosynthesis of nerolidol.

In order to solve the technical problems mentioned above, the present application proposes the following technical solutions:

The first aspect of the present application provides a nerolidol synthase comprising the domains of Pfam Nos. PF01397 and PF03936, and having the activity of nerolidol synthase.

The second aspect of the present application provides a polynucleotide molecule comprising at least one of the nucleotide sequences encoding the nerolidol synthase in the first aspect of the present application or the complementary sequence thereof.

The third aspect of the present application provides a nucleic acid construct comprising at least one of the polynucleotide molecules provided in the second aspect of the present application.

The fourth aspect of the present application provides a recombinant strain comprising the polynucleotide molecule provided in the second aspect of the present application, or the nucleic acid construct provided in the third aspect of the present application.

The fifth aspect of the present application provides the use of nerolidol synthase in the first aspect of the present application, the polynucleotide molecule in the second aspect of the present application, the nucleic acid construct in the third aspect of the present application or the recombinant strain in the fourth aspect of the present application in the production of nerolidol.

The sixth aspect of the present application provides a method for preparing nerolidol, comprising biosynthesizing nerolidol using the recombinant strain in the fourth aspect of the present application.

The present application identifies a novel nerolidol synthase and the encoding gene thereof, and the encoding gene of the nerolidol synthase is integrated into a nucleic acid construct and introduced into a host cell to obtain a recombinant strain, so that the encoding gene is expressed in the recombinant strain, and in turn the biosynthesis of nerolidol is realized. By adopting the recombinant strain and the method for biosynthesizing nerolidol of the present application, the yield of nerolidol is remarkably improved.

### Brief Description of the Drawings

FIG. 1 shows a schematic diagram of the construction of plasmid pZY900;
FIG. 2 shows a schematic diagram of the construction of plasmids pYR013, pYR007, pArar-TPS27, pArar-TPS28, pYR006, pYR010, pCaNES2, pCaNES1, pTwNES, pAcNES1, pFaNES1, pCsNES2 and pLpNES1;
FIG.3 is the extracted ion flow chromatogram of nerolidol characteristic ion m/z=93 in the fermentation products of strains CCJ-1 and S900;
FIG.4 is the mass spectrum of nerolidol in the fermentation products of strains CCJ-1 and S900;
FIG.5 is the extracted ion flow chromatogram of nerolidol characteristic ion m/z=93 in the fermentation products of strains LXF-1, LXF-1-1, LXF-1-2 and S900;
FIG.6 is the mass spectrum of nerolidol in the fermentation products of strains LXF-1, LXF-1-1, LXF-1-2 and S900;
FIG.7 is the extracted ion flow chromatogram of nerolidol characteristic ion m/z=93 in the fermentation products of strains AH-1, AH-2 and S900;
FIG. 8 is the mass spectrum of nerolidol in the fermentation products of strains AH-1, AH-2 and S900;
FIG. 9 shows a schematic diagram of the construction of plasmids pYR020, pYR021, pYR017 and pYR018;
FIG.10A shows a schematic diagram of the construction of plasmid pYH395;
FIG.10B shows a schematic diagram of the knockout element construction of the upstream activation *cis* element (-220 to-175) of the ERG9 promoter;
FIG.11 is the schematic diagram of the construction of pZY521 knockout cassette;
FIG.12 shows the shake flask fermentation yields of strains comprising nerolidol synthase CCJ_TPS23;
FIG.13 shows the shake flask fermentation yields of strains comprising nerolidol synthase ACH_TPS07.

### Detailed Description of the Embodiments

The terms and descriptions used herein are only for the purpose of describing particular embodiments and are not intended to limit the application. Unless otherwise defined, all technical and scientific terms used herein have the same meaning as commonly understood by one of ordinary skill in the art to which this disclosure pertains. In addition, unless the context requires otherwise, singular terms shall include the plural and plural terms shall include the singular.

### Definition

As used herein, the terms "a" and "an" and "the" and similar references indicate both singular and plural unless otherwise indicated herein or clearly contradicted with the context.

As used herein, the terms "about" and "similar to" mean within an acceptable error range for a particular value as determined by one of ordinary skill in the art, which may depend in part on how the value is measured or determined, or on the limitations of the measurement system.

The term "nucleic acid" or "polynucleotide" refers to deoxyribonucleic acid (DNA) or ribonucleic acid (RNA) and polymers thereof in single or double stranded form. Unless explicitly limited, the term "nucleic acid" or "polynucleotide" also includes nucleic acids comprising known analogs of natural nucleotides that have binding properties similar to those of the reference nucleic acid and are metabolized in a manner similar to naturally occurring nucleotides (see, Kariko et. al. U.S. Patent No. 8278036, which discloses mRNA molecules in which uridine is replaced by pseudo uridine, methods of synthesizing the mRNA molecules, and methods for delivering therapeutic proteins *in vivo*)*.* Unless otherwise indicated, a particular nucleic acid sequence also implicitly includes conservatively modified variants thereof (e.g., degenerate codon substitutions), alleles, orthologs, single nucleotide polymorphisms (SNPs), and complementary sequences, as well as explicitly indicated sequences.

"Construct" refers to any recombinant polynucleotide molecule (e.g., plasmid, cosmid, virus, autonomously replicating polynucleotide molecule, phage, linear or circular single or double stranded DNA or RNA polynucleotide molecule), which may be derived from any source, capable of integrating with the genome or autonomously replicating, which may be operably linked to one or more polynucleotide molecules. In the present application, constructs generally comprise a polynucleotide molecule of the present application operably linked to transcription initiation regulatory sequences that direct the transcription of the polynucleotide molecule of the present application in a host cell. Heterogeneous promoters or endogenous promoters may be used to direct expression of the nucleic acids of the present application.

"Vector" refers to any recombinant nucleic acid construct that can be used for transformation purposes (i.e., introduction of heterologous DNA into a host cell). The vector may comprise a resistance gene for growth in an organism and a promoter for expression of the protein of interest in the organism. Certain vectors are capable of autonomous replication in the host cell into which they are introduced (e.g., vectors having an origin of replication that functions in the host cell). Other vectors may be introduced into the host cell and integrated into the genome of the host cell and thus replicated together with the host genome. Furthermore, certain preferred vectors are capable of directing the expression of foreign genes to which they are linked. One type of vector is a "plasmid", which generally refers to a circular double-stranded DNA loop into which additional DNA segments (exogenous genes) may be ligated, and may also include linear double-stranded molecules, such as those obtained from amplification by polymerase chain reaction (PCR) or treatment of circular plasmids with restriction enzymes.

Plasmid vectors comprise vector backbones (i.e. empty vectors) and expression frameworks.

The term "expression framework" refers to sequences having the potential to encode proteins.

The term "host cell" refers to a cell capable of introducing a gene of interest and providing conditions for cloning and/or expression of the gene of interest, such as a microorganism, and specifically may be a bacterium (such as *Escherichia coli*), a yeast (such as *Saccharomyces cerevisiae*), an actinomycete, or the like.

The term "recombinant strain" refers to genetically engineered strains (such as bacteria, yeasts, actinomycetes, etc.), which means that exogenous gene fragments have been introduced into the strain, wherein one way of modification includes the introduction of new DNA fragments into the genome of the strain to cause a change, and the other way includes the introduction of artificially constructed or modified plasmids into the strain so that the strain acquires the ability to express the target gene.

The first aspect of the present application provides a nerolidol synthase comprising the domains of Pfam Nos. PF01397 and PF03936, and having the activity of nerolidol synthase.

**In** some embodiments, the nerolidol synthase is from *Tanacetum cinerariifolium, Astilbe chinensis*, or *Artemisia argyi.*

**In** some embodiments, the nerolidol synthase has the amino acid sequence having at least 90%, 91%, 92%, 93%, 94%, 95%, 96%, 97%, 98%, 99% or 100% sequence identity to the amino acid sequence as shown in SEQ ID NO. 1, SEQ ID NO. 2, SEQ ID NO. 3, SEQ ID NO. 4, SEQ ID NO. 11 or SEQ ID NO. 12.

**In** some embodiments, the nerolidol synthase has the following amino acid sequences (amino terminal to carboxy terminal):
CCJ_TPS23 (*Tanacetum cinerariifolium* source) 
MIINNCVTPKDTQPAPLPSETDIVSNDINIKNELSLKHLELLEEVRYLLKNYSSEPLDMVDA
LQKLCINHYYEDEIGLILKLLYARMSNNGDYKHDKSLYEVSLSFRILRQEGYYVSADVFADFKQK
DGKFKEEIAQDVKGLMALYEASQLSLDGERILEEASDFSSGALKDMVPSLDLHQAMIATNTLQHT
YQRTSSTFMVKKFMKVYTGTPMCELAQLELTTKVQSLHRTELEQISRWWKDLGLAQELKLARN
 
QPLHWYLWPMASLTDLSLSEQRIELTKPIAFIFLIDDIFDVYGTLDQLVILTQAVNRWESNSLERLP
HHLRICIQALFDVTNEISDNIYKKHGFNPIDFLKQSWINLCEAFLVEAKWFAEGYMPTAEDYLNN
GMVSTGAHVVIVHMFFLLGGGPNIKSASVVNENQGITSCLAKILRLWDDLGSAEDVDQDGNDGS
YVTYYMKENAGCSIQKAHEHVMEMISNTWKQLNAECLYSSHLSRTFTKACLNLARMIPMMYDY
DENHSLPFIKEYINSMF (SEQ ID NO.1)
ACH-TPS07 (*Astilbe chinensis* source) 
MAPPPSSYPPSARNNFTQVGTGLSDTMNEPSTQKWSITSHDGTLVSSPIILHNSKANNACY
TDEFHVDHESKLNEVRDLLNKVGEDYQLEGLVMIDAIQCLNIDYRFRKEIESILQSQDETSRVHEN
DEHDLYQTALRFRLLRQEGHFVSAEVFDKFKNKEGKFKQELVEDIRGMMGLYEAAQLRIEGEHIL
DEAESFCRRLFKGCMPYLNSHEAKLVESTLKHPYRKSLSRIGAKNFANNFQGNFKWITVLEELSN
LDLKIVQSIHQKELQQISHWWEELGLAKELKLARDQPLKWHMWSMEVLQDPSLSEQRIELTKPIS
LVYIIDDIFDVYGTLDELTLFTEAVTRWEFTDANQLPNYMRTCFQTLFDITYEIGYKFFIVHGWNPI
DTLRKSWVTLCKAFLAEAKWFASGELPKADKYLKNGIISSGVHVVLVHMFFLLGQDLTKECLDL
VNDNNNIPGIINYTATILRLWDDLGSAKDENQDGYDGSYLECFMNEHKGLLMKTAREHMIGMIS
DAWKCLNKECLSPNPFSSSFTKASLNAARMVPLMYNYDDNHCLPDLEEHIQCLF (SEQ ID NO.2)
Arar-TPS27 (*Artemisia argyi* source) 
MTISVTRKDTQPTPFPSETDIVSNDINIKNELSIRHLELLEEVRNLLKNCSSKPLVMVDTLQ
KLCINHHYEEEIGLILKSLYTRMSNNDDYKHDKSLYEVSLSFRILRQHGYYVSADVFANFKQKDG
KFKGEITQDLKGLIALYEASQLSLEGEQILKEASDFSSGALKEMMPSLDQDQAMIVTNTLQHTYQ
RTTSTFMVKKFMKVYTGTPMCELAQLELTKVQSLHRTEVDQISRWWKGLGLAQELKLARNQPL
HWYLWPMASLTDLSLSEQRVELTKPIAFIFLIDDIFDVYGTLDQLVILTQAVNRWESNRLEQLPYHL
RICIQALFDVTNEISDKIYKKHGFNPIEFLKQSWINLCDAFLVEAKWFAEGYMPTAEDYLNNGMV
STGVHVVIVHMFFLLGGGPNIKSASVVNENQGIMSCLAKILRLWDDLGSAKDVDQDGNDGSYV
TYYMKENAGCSIQKAHEHVMEMISNTWKQLNAECLYSSHFSRTFTKACLNLAKMIPMMYDYDE
NHSLPFIEEYINSMF (SEQ ID NO.3)
Arar-TPS28 (*Artemisia argyi* source) 
MSINILHGDLPDVKVMSPQADATEKIDELKEKIRRVLMTTSDPKMSLKLVDTIQRLRIGYY
FQEDINEILEKLKQCLPDDELHIVALCFRLLRQNGIPTNSEVFRKFIDMNGEFIKSTSEDIEGLLSLY
EASYMGSNEEIFLVHAKKITTRELNICVPKLSPKLSKKVLQALELPMHLRMETLEARRYIEDYGNE
EDHNPLLLELAKLDYNHVQSLFRRELVEMARWWNHLGIARKFSFVRDRHVECFLWTVGVLPEP
KYSATRIVMAKITSILLLLDDIYDTYGSYDDLVLLTKIIQRWDMTEIEQLPEYMQACYMALYNTTS
EICDKVLRERGLYVEQFLRKTWIKIVEGYMVEVKWLKTGTIPNFKEYMDNAVTTSGSYMAFVH
MFFLICDEVNKENMADLLEPYPKFFTLAGTILRLWDDLGTVKEEQERGEVLSSIQLLMKEKKITC
DKDERKQILELIHELWKDLNAELVAPNAVLWPMIRVALNMSRTSQVVYQHNEDSYLSSVKDHVK
NLFFKAIDM (SEQ ID NO.4)
ACH-TPS08 (*Astilbe chinensis* source) 
MAPPPSSYPPSARNNFTQVGTGLGDTMNEPSTQKWSITSHDGTLVSSPIILHNSKANNACY
TDEFHVDHERKLNEVRDLLNKVGEDYQLEGLVMIDAIQCLNIDYHFRKEIESILQCQDETSRVHE
NDEHDLYQTALRFRLLRQEGHFVSAEVFDKFKNKEGKFKQELVEDIRGMMGLYEAAQLRIEGEH
ILDEAESFCRRLFKACMPYLNSHEARLVESMIKHPYRKSLSRIGAKNFANNFQGNFKWITVLEELS
NLDLKIVQSIHQKELQQISHWWEELGLAKELKLARDQPLKWHMWSMEVLTDPSLSEQRIELTKPI
SLVYIIDDIFDVYGTLDELTLFTEAVTRWEFTDVNQLPNYMRTCFQTLFDITYEIGYKFFIVHGWNP
INTLRKSWVTLCKAFLAEAKWFASGELPKADKYLKNGIISSGVHVVLVHMFFLLGQDLTKECLDL
VNDNNNIPGIINYTATILRLWDDLGSAKDENQDGYDGSYLECFMNEHKGLLMKTAREHMIGMIS
DAWKCLNKECLSPNPFSSSFTKASLNAARMVPLMYNYDDNHCLPDLEEHAMFVLNMINVT
(SEQ ID NO.11)
ACH-TPS09 (*Astilbe chinensis* source)

MAPPPSSYPPSARNNFTQVGTGLGDTMNEPSTQKWSITSHDGTLVSSPIILHNSKANNACY
TDEFHVDHERKLNEVRDLLNKVGEDYQLEGLVMIDAIQCLNIDYHFRKEIESILQCQDETSRVHE
NDEHDLYQTALRFRLLRQEGHFVSAEVFDKFKNKEGKFKQELVEDIRGMMGLYEAAQLRIEGEH
ILDEAESFCRRLFKACMPYLNSHEARLVESMIKHPYRKSLSRIGAKNFTNNFQGNFKWITVLEELS
NLDLKIVQSIHQKELQQISHWWEELGLAKELKLARDQPLKWHMWSMEVLTDPSLSEQRIELTKPI
SLVYIIDDIFDVYGTLDELTLFTEAVTRWEFTDVNQLPNYMRTCFQTLFDITYEIGYKFFIVHGWNP
IDTLRKSWVTLCKAFLAEAKWFASGELPKADKYLKNGIISSGVHVVLVHMFFLLGQDLSKECLDL
VNDNNNIPGIINYTATILRLWDDLGSAKDENQDGYDGSYLECFMNEHKGLLMKTAREHMIGMIS
DAWKCLNKECLSPNPFSSSFTKASLNAARMVPLMYNYDDNHCLPDLEEHAMFVLNMINVT
(SEQ ID NO.12).

The second aspect of the present application provides a polynucleotide molecule comprising at least one of the nucleotide sequences encoding the nerolidol synthase in the first aspect of the present application or the complementary sequence thereof.

In some embodiments, the polynucleotide molecule comprises the nucleotide sequence having at least 90%, 91%, 92%, 93%, 94%, 95%, 96%, 97%, 98%, 99% or 100% sequence identity to the nucleotide sequence as shown in SEQ ID NO.5, SEQ ID NO.6, SEQ ID NO.7, SEQ ID NO.8, SEQ ID NO.9 or SEQ ID NO.10.

In some embodiments, the polynucleotide molecule comprises the nucleotide sequences (5' end to 3' end) as shown below:
CCJ_TPS23 (*Tanacetum cinerariifolium* source) 
atgatcatcaacaattgtgttacaccgaaagacacccagccagcccctctcccatctgaaacagatattgtgtcaaatgacattaacattaagaa
tgaattgtccctcaaacatctagaattgttagaagaagtccgatatcttctaaagaattattcttccgaacctttggacatggttgacgctcttcaaaaactttgc
atcaaccattactatgaagacgagattggcttgattttgaaattgctctacgctagaatgtcgaataatggcgactacaagcatgataaaagtctttatgaggt
ttccctttcttttcgcatccttcgtcaagaaggttactatgtctcagctgatgtttttgctgatttcaaacaaaaggatgggaagtttaaagaagaaatagcacaa
gatgttaagggtctaatggcgttatatgaagcttcacagctaagcctggatggagaacgaatacttgaagaggcttcagattttagcagtggtgctcttaaa
 
gacatggtgccttctttagatctacatcaagctatgattgctactaacacattacaacatacgtatcaaagaacctcatcaactttcatggttaaaaaattcatga
aagtatacacgggtacacccatgtgtgaactagcccaattagagttgactactaaagtccagtcactacatcgaacagaactagaacaaatctccagatgg
tggaaggacttggggttggcgcaagagttgaagcttgcaagaaaccaaccgttgcactggtacttatggccaatggctagcctcacagatctcagcttgtc
cgagcagagaattgagctcacgaagcccattgctttcatctttctaattgatgacatttttgatgtttatggaactctagaccaacttgttatcttgacacaagca
gtgaatagatgggaaagtaacagccttgaacgactcccacatcacctaaggatctgtattcaagctctatttgacgtaacaaacgaaataagtgacaatatc
tacaagaagcatgggttcaatcccattgattttttgaaacaatcgtggataaacctttgtgaagcttttctagtagaggcgaaatggtttgctgagggatacat
gccgacggcagaggattacttgaataatgggatggttagtacaggggcacatgtcgtgatagtgcacatgttcttcctcctcggtggtggccctaacataa
aaagtgctagcgttgtaaacgaaaaccaagggattacgtcttgtttggcaaagattcttcgtctttgggatgacttaggaagtgctgaggatgtggatcaag
acggtaatgatggatcatatgtgacatattacatgaaagagaatgcaggttgttctattcaaaaggcacatgaacatgttatggaaatgatatcgaatacttg
gaagcaactaaatgcggagtgcctttactcaagtcatttatcacgtacattcaccaaagcttgcctcaatcttgcaagaatgattccaatgatgtacgattatg
atgagaatcattcccttcctttcatcaaggaatatataaactccatgttttaa (SEQ ID NO.5)
ACH-TPS07 (*Astilbe chinensis* source) 
atggcaccccctccttcctcctatcctccaagtgctcgaaacaattttacacaagttggcacaggacttagtgatacgatgaacgagccttctac
tcagaaatggagcattaccagccatgatggcaccttagtttcaagccctataatacttcacaactctaaagcaaacaatgcttgttatactgatgaatttcatgt
cgatcatgaaagcaaattgaatgaagttagggatttgcttaacaaagttggagaagattatcaattagaaggtttggtcatgatcgacgccattcaatgccta
aacattgactatcgcttccgaaaggagattgagtccatcctacaaagtcaggatgagacatctagggttcatgaaaatgatgaacacgatctttaccagact
gcacttcgttttcgactgctgagacaagaaggccattttgtgtctgcagaagtgtttgacaaattcaagaacaaagaggggaagtttaagcaagaactagtt
gaagatataaggggaatgatgggcttatatgaagctgcgcagcttagaattgaaggagaacatatacttgatgaagccgaaagcttttgtcgtcggctcttt
aaaggatgcatgccctatcttaactctcatgaagcaaaacttgtcgagagcacgctaaagcatccctatcgaaagagcttgtcacgcataggggccaaaa
acttcgctaacaatttccaaggcaattttaaatggattactgttcttgaagaactatcaaatttagatttgaaaatagttcaatctatacaccagaaggaactaca
acaaatttcccattggtgggaagaacttggtttagcaaaggaattgaagctcgcaagagaccaaccgctaaaatggcacatgtggtccatggaagtgttac
aagatccaagcttgtctgagcaaaggattgagcttacaaagcccatttctcttgtatacataattgatgacattttcgacgtttatgggacgcttgatgaactca
ctctcttcacggaagcagtcactagatgggaattcaccgacgcaaatcaactgcctaactacatgaggacatgtttccagactctctttgatatcacttatgaa
attggctacaagtttttcatagttcatggttggaaccctatagacacactacgaaaatcgtgggttactttgtgcaaagcatttttggcggaagcaaaatggttt
gcttctggggaattgccaaaggcagataaatacttgaaaaatggaattattagttcaggagtgcatgtagtacttgttcacatgttttttctcttgggtcaagatt
taacaaaggaatgtttagacctagtgaacgacaacaacaatattccaggcatcataaactatacggcaacgattcttcgtctttgggatgacttgggaagtg
caaaggatgagaatcaagatgggtacgacggatcatatctggaatgtttcatgaatgaacacaaaggtttattaatgaagactgcaagagagcatatgatt
ggcatgatttcggatgcatggaagtgcttgaacaaggaatgcctctctccaaatccattttcatcatctttcacaaaggcttctcttaatgctgcaaggatggtt
ccattgatgtacaattacgacgacaaccattgtctcccagatcttgaggagcatatacaatgtttgttttaa (SEQ ID NO.6)
Arar-TPS27 (*Artemisia argyi* source) 
atgacaatctctgttactagaaaggatactcaaccaacaccatttccatctgaaactgatattgtttctaacgatatcaacatcaagaacgaattgt
ctattagacatttggaattgttggaagaagttagaaatttgttgaagaactgttcttctaagccattagttatggttgatactttacaaaagctgtgtattaaccatc
attacgaagaagaaatcggtttgattttgaaatctttgtacacaagaatgtccaataacgatgattataagcatgataagtccttatacgaagtttctttgtctttta
gaatcttgagacaacatggttattacgtttctgctgatgtttttgctaattttaagcaaaaggacggtaaatttaagggtgaaattacacaagatctgaaaggttt
aatcgctttatatgaagctagtcaattgtctttggaaggtgaacaaattttaaaggaagctagtgatttctcttctggtgctttgaaagaaatgatgccatctttg
gatcaagatcaagctatgattgttacaaatactttgcaacatacctaccaaagaacaacttctacttttatggttaagaagttcatgaaggtttacactggtaca
ccaatgtgtgaattggctcaattggaattgactaaagttcaatctttgcatagaacagaagttgatcaaatttctagatggtggaaaggtttaggtttggctcaa
 
gaattaaaattagctagaaaccaaccattgcattggtatttatggccaatggcttctttgactgatttgtctttgtctgaacaaagagttgaattgactaagccaa
ttgcttttattttcttgatcgatgatatcttcgacgtttatggtactttggatcaattagttatcttgactcaagctgttaatagatgggaatctaatagattagagca
attaccataccatttgagaatttgtatccaagctctatttgatgttacaaatgaaatctctgacaagatttacaagaagcatggttttaatccaatcgaatttttgaa
gcaatcctggattaatttgtgtgatgctttttggttgaggctaaatggtttgctgaaggttatatgccaactgctgaagattatttaaacaatggtatggtttcca
caggtgttcatgttgttattgttcatatgtttttcctgttgggtggtggtccaaatattaaatctgcttctgttgttaacgagaatcaaggtattatgtcttgtttggct
aaaattttgaggttatgggatgatttaggttctgctaaagatgttgatcaagatggtaatgatggttcttatgttacatattacatgaaggaaaacgctggttgtt
ctattcaaaaagctcatgaacatgttatggaaatgatttctaacacttggaaacaattgaacgctgaatgtttatattcctctcatttttctaggacattcactaaa
gcatgtttgaatttggctaaaatgatcccaatgatgtatgattatgacgaaaatcattccttgccatttattgaagaatacattaactccatgttctaa (SEQ
ID NO.7)
Arar-TPS28 (*Artemisia argyi* source) 
atgtctatcaacatcttgcatggtgatttgccagatgttaaagttatgtctccacaagctgatgctacagaaaaaattgatgaattaaaggagaag
atcaggagagttttgatgactacatctgatccaaaaatgtctttaaagttggttgatactatccaaagattgagaattggttattacttccaagaagatatcaac
gaaatcttggaaaaattgaagcaatgtttgccagatgatgaattgcatattgttgctttgtgttttagattgttgagacaaaatggtatcccaacaaattctgaag
tttttagaaagttcatcgacatgaatggtgaatttattaagtctacttccgaagatatcgaaggtttgttatctttgtatgaagctagttatatgggttctaatgaag
aaattttcctggttcatgctaaaaaaatcacaactagagaattgaacatctgtgttccaaaattgtctccaaaattgtcaaaaaaggtcttgcaagctctagaat
tgccaatgcatttgagaatggaaacattggaagctagaagatatattgaagattacggtaatgaggaagatcataatccattattactggaattggctaaatt
ggattacaatcatgttcaatctctgtttagaagagaattagttgaaatggctagatggtggaatcatttgggtattgctagaaaattttcctttgttagagacaga
catgttgaatgttttttgtggactgttggtgttttgccagaaccaaaatattctgctactagaattgttatggctaaaattacttccatcttgttattgttggacgatat
ttatgacacttacggttcttatgatgatttggttttattgacaaagatcatccaaagatgggatatgacagaaattgaacaattgccagaatatatgcaagcatg
ttatatggctttgtataatactacatccgaaatttgtgacaaggttttaagagaaagaggtttgtatgttgaacaatttttgagaaagacctggattaaaatcgttg
aaggttatatggttgaggttaaatggttaaaaaccggtactattccaaattttaaggaatatatggacaacgctgttactacttctggttcttatatggcttttgttc
atatgtttttcctgatttgtgacgaagttaataaggaaaatatggctgatttgttggaaccatatccaaaatttttcactttggctggtacaattttgagattgtggg
atgatttaggtactgttaaagaagaacaagaaagaggtgaagttttgtcttctattcaattattgatgaaggagaagaagatcacttgtgataaagatgaaag
aaagcaaatcttggaattgattcatgaattgtggaaagatttgaacgctgaattagttgctccaaatgctgttttgtggccaatgattagagttgctttaaatatg
tctaggacatctcaagttgtttatcaacataatgaggattcttacttgtcttctgttaaagatcatgttaagaacttgttcttcaaggctattgatatgtaa (SEQ
ID NO. 8)
ACH-TPS08 (*Astilbe chinensis* source) 
atggcaccccctccttcctcctatcctccaagtgctcgaaacaattttacacaagttggcacaggacttggtgatacgatgaacgagccttctac
tcagaaatggagcattaccagccatgatggcaccttagtttcaagccctataatacttcacaactctaaagcaaacaatgcttgttatactgatgaatttcatgt
cgatcatgaaagaaaattgaatgaagttagggatttgcttaacaaagttggagaagattatcaattagaaggtttggtcatgatcgacgccattcaatgccta
aacattgactatcacttccgaaaggagattgagtccatcctacaatgtcaggatgagacatctagggttcatgaaaatgatgaacacgatctttaccagact
gcacttcgttttcgactgctgagacaagaaggccattttgtgtctgcagaagtgtttgacaaattcaagaacaaagaggggaagtttaagcaagaactagtt
gaagatataaggggaatgatgggcttatatgaagctgcgcagcttagaattgaaggagaacatatacttgatgaagccgaaagcttttgtcgtcggctcttt
aaagcatgcatgccctatcttaactctcatgaagcaagacttgtcgagagcatgataaagcatccctatcgaaagagcttgtcacgcataggggccaaaaa
cttcgctaacaatttccaaggcaatttcaaatggattactgttcttgaagaactatcaaatttagatttgaaaatagttcaatctatacaccagaaggaactacaa
caaatttcccattggtgggaagaactgggtttagcaaaggaattgaagctcgcaagagaccaaccgctaaaatggcacatgtggtccatggaagtgttaa
cagatccaagcttgtctgagcaaaggattgagcttacaaagcccatttctcttgtatacataattgatgacattttcgacgtttatgggacgcttgatgaactca
 
ctctcttcacggaagcagtcacaagatgggaattcaccgacgtaaatcaactgcctaactacatgaggacatgtttccagactctctttgatatcacttatgaa
attggctataagtttttcatagttcatggttggaaccctataaacacactacgaaaatcgtgggttactttgtgcaaagcatttttggcggaagcaaaatggttt
gcttctggggaattgccaaaggcagataaatacttgaaaaatgggattattagttcaggagtgcatgtagtacttgttcacatgttttttctcttgggtcaagatt
taacaaaggaatgtttagacctagtgaacgacaacaacaatattccaggcatcataaactatacggcaacgattcttcgtctttgggatgacttgggaagtg
caaaggatgagaatcaagacgggtacgacggatcatatctggaatgtttcatgaatgaacacaaaggtttattaatgaagactgcaagagagcatatgatt
ggcatgatttcggatgcatggaagtgcttgaacaaggaatgcctctctccaaatccattttcatcatctttcacaaaggcttctcttaatgctgcaaggatggtt
cctttgatgtacaattacgacgacaaccattgtctcccagatcttgaggaacatgcaatgtttgttttaaatatgataaacgttacatag (SEQ ID NO.9)
ACH-TPS09 (*Astilbe chinensis* source)

atggcaccccctccttcctcctatcctccaagtgctcgaaacaattttacacaagttggcacaggacttggtgatacgatgaacgagccttctac
tcagaaatggagcattaccagccatgatggcaccttagtttcaagccctataatacttcacaactctaaagcaaacaatgcttgttatactgatgaatttcatgt
cgatcatgaaagaaaattgaatgaagttagggatttgcttaacaaagttggagaagattatcaattagaaggtttggtcatgatcgacgccattcaatgccta
aacattgactatcacttccgaaaggagattgagtccatcctacaatgtcaggatgagacatctagggttcatgaaaatgatgaacacgatctttaccagact
gcacttcgttttcgactgctgagacaagaaggccattttgtgtctgcagaagtgtttgacaaattcaagaacaaagaggggaagttcaagcaagaactagtt
gaagatataaggggaatgatgggcttatatgaagctgcgcagcttagaattgaaggagaacatatacttgatgaagccgaaagcttttgtcgtcggctcttt
aaagcatgcatgccctatcttaactctcatgaagcaagacttgtcgagagcatgataaagcatccctatcgaaagagcttgtcccgcataggggccaaaaa
cttcactaacaatttccaaggcaatttcaaatggattactgttcttgaagaactatcaaatttagatttgaaaatagttcaatctatacaccagaaggaactacaa
caaatttcccattggtgggaagaactgggtttagcaaaggaattgaagctcgcaagagaccaaccgctaaaatggcacatgtggtccatggaagtgttaa
cagatccaagcttgtctgagcaaaggattgagcttacaaagcccatttctcttgtatacataattgatgacattttcgacgtttatgggacgcttgatgaactca
ctctcttcacggaagcagtcacaagatgggaattcaccgacgtaaatcaactgcctaactacatgaggacatgtttccagactctctttgatatcacttatgaa
attggctacaagtttttcatagttcatggttggaaccctatagacacactacgaaaatcgtgggttactttgtgcaaagcatttttggcggaagcaaaatggttt
gcttctggggaattgccaaaggcagataaatacttgaaaaatgggattattagttcaggagtgcatgtagtacttgttcacatgttttttctcttgggtcaagatt
tatcaaaggaatgtttagacctagtgaacgacaacaacaatattccaggcatcataaactatacggcaacgattcttcgtctttgggatgacttgggaagtgc
aaaggatgagaatcaagacgggtacgacggatcatatctggaatgtttcatgaatgaacacaaaggtttattaatgaagactgcaagagagcatatgattg
gcatgatttcggatgcatggaagtgcttgaacaaggaatgcctctctccaaatccattttcatcatctttcacaaaggcttctcttaatgctgcaaggatggttc
ctttgatgtacaattacgacgacaaccattgtctcccagatcttgaggaacatgcaatgtttgttttaaatatgataaacgttacatag (SEQ ID NO.10).

The third aspect of the present application provides a nucleic acid construct comprising at least one of the polynucleotide molecules provided in the second aspect of the present application. In the present application, the polynucleotide molecule ligated to the nucleic acid construct is referred to as the target gene, and the enzyme encoded by the polynucleotide molecule is referred to as the target protein.

**In** some embodiments, the nucleic acid construct further comprises regulatory elements for regulating the expression of the target gene, such as promoters, terminators, etc. Exemplary promoters may be constitutive promoters such as P_{TEF1}, P_{TDH3}, P_{GPM1}, P_{TPI1}, etc., inducible promoters such as P_{HXT1} (induced by high concentration of glucose), P_{CUP1} (induced by copper ions), P_{GAL1}, P_{GAL2}, P_{GAL7}, P_{GAL10} (induced by galactose), etc. Those skilled in the art may select as needed, and is not limited herein by the present application.

In some embodiments, the nucleic acid construct further comprises a marker gene for screening recombinant strains comprising a target gene or target protein, such as leucine screening marker, histidine screening marker, tryptophan screening marker, uracil screening marker, etc., which can be specifically selected by a person skilled in the art as needed, and is not limited herein by the present application.

In some embodiments, the nucleotide sequence is located between two insertion elements, wherein the insertion elements are for integrating the nucleotide sequence into the genome of a host cell.

In some embodiments, a nucleotide sequence having insertion elements linked at both ends is ligated to a nucleic acid construct, such as a plasmid backbone of a plasmid vector, and when the nucleic acid construct is used for introducing a target gene into a host cell, the nucleic acid construct can be digested by tools such as a restriction enzyme to obtain a linearized target gene fragment having insertion elements linked at both ends thereof, and the linearized target gene fragment can be introduced into the host cell, so that it can be inserted into the corresponding position of host cell genome through insertion elements at both ends, thus obtaining the recombinant strain of the present application.

A person skilled in the art can introduce the linearized target gene fragment into the host cell by conventional methods, for example, lithium acetate method can be used for yeast, calcium transformation method can be used for *Escherichia coli*, etc., which are conventional operations in the art, and is not limited herein by the present application.

In some embodiments, the two insertion elements appear in pairs, for example, they can be the left and right homologous arms of Leu2, the left and right homologous arms of Ura3, the left and right homologous arms of YPRCdelta15, etc. The homologous arms of different genes can integrate the gene of interest into different locations in the host cell genome. Those skilled in the art can specifically select the type of homologous arms according to the location they expect to be integrate into in the host cell genome, and is not limited herein by the present application.

In some embodiments, regulatory elements such as promoters and terminators for regulating the expression of the target gene are further included between the two insertion elements. The types of the promoters and terminators are not limited by the present application.

In some embodiments, the nucleic acid construct further comprises a nucleotide sequence which encodes at least one selected from acetoacetyl coenzyme A thiolase (ERG10), hydroxy-methylglutaryl coenzyme A synthase (ERG13), hydroxy-methylglutaryl coenzyme A reductase (HMG1), mevalonate kinase (ERG12), mevalonate-5-phosphate kinase (ERG8), mevalonate pyrophosphate decarboxylase (MVD1), isoprene pyrophosphate isomerase (IDI1), farnesyl pyrophosphate synthase (ERG20); wherein the names of the genes encoding these enzymes are shown in parentheses.

In some embodiments, the hydroxy-methylglutaryl coenzyme A reductase is a truncated hydroxy-methylglutaryl coenzyme A reductase (tHMG1), and the endoplasmic reticulum localization sequence is truncated in tHMG1, thus enhance the stability of the enzyme in the cytoplasm.

Exemplary but non-limiting disclosures of the genes encoding the above enzymes are as follows:
ERG10 (Accession/GENE ID: 856079), ERG13 (Accession/GENE ID: 854913), tHMG1 (Accession/GENE ID: 854900, 4-1659bp truncated), ERG12 (Accession/GENE ID: NM_001182715.1), ERG8(Accession/GENE ID: CP046093.1, 689693..691048), MVD1 (Accession/GENE ID: NM_001183220.1), IDI1 (Accession/GENE ID: NM_001183931.1), ERG20 (Accession/GENE ID: 853272).

The inventors have found that acetoacetyl coenzyme A thiolase, hydroxy-methylglutaryl coenzyme A synthase, hydroxy-methylglutaryl coenzyme A reductase, mevalonate kinase, mevalonate-5-phosphate kinase, mevalonate pyrophosphate decarboxylase, and isoprene pyrophosphate isomerase belong to enzymes in the mevalonate pathway, and in the mevalonate pathway isoprene pyrophosphate (IPP) and dimethylallyl pyrophosphate (DMAPP) can be synthesized, both of which can be used as precursors for synthesizing farnesyl pyrophosphate (FPP) under the catalysis of farnesyl pyrophosphate synthase, whereas FPP is the substrate in the biosynthesis of nerolidol, thus when at least one of the enzymes in the mevalonate pathway and the farnesyl pyrophosphate synthase is included in the nucleic acid construct, the synthesis of FPP would be facilitated, and in turn the biosynthesis of valencene would be facilitated.

**In** some embodiments, the nucleic acid construct is a plasmid vector; preferably, the plasmid vector is a eukaryotic expression vector.

**In** some embodiments, the nucleic acid construct comprises a pRS426 plasmid backbone. The inventors have found that the pRS426 plasmid backbone comprises *AmpR* screening marker suitable for *Escherichia coli, URA3* screening marker suitable for *Saccharomyces cerevisiae,* and replicon suitable for *Escherichia coli* and multiple copy replicon suitable for *Saccharomyces cerevisiae,* and the use of the pRS426 plasmid backbone facilitates the maintenance of high copies of the plasmid comprising the gene of interest after introduction into *Saccharomyces cerevisiae.*

In some embodiments, a mutation is present in the pRS426 plasmid backbone, wherein the mutation eliminates the cleavage site BsaI in the pRS426 plasmid backbone, thereby allowing BsaI to be used as a restriction enzyme when constructing vectors using the Goldengate method.

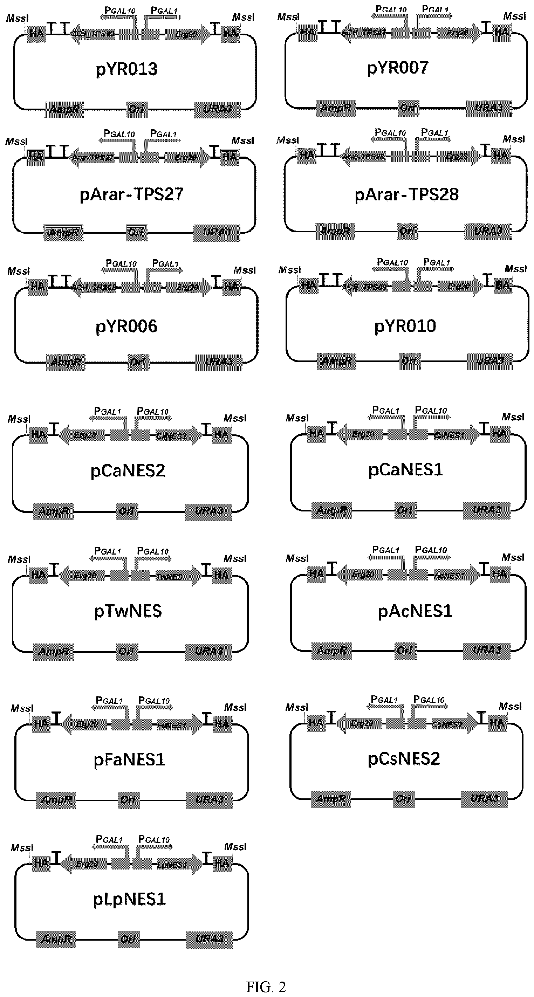

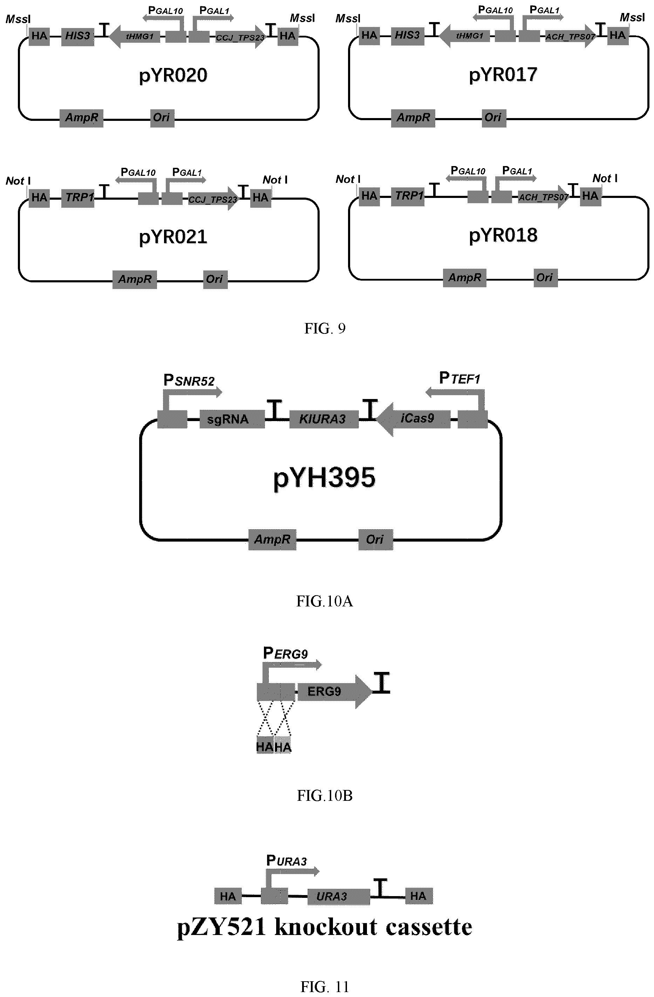

In some embodiments, the nucleic acid construct is at least one of plasmid vectors pYR006, pYR007, pYR010, pYR013, pAra-TPS27, pAra-TPS28, pYR017, pYR018, pYR020, and pYR021; and the construction schematic diagram of the plasmid vectors is as shown in FIG. 2 or FIG. 9.

In some embodiments, the plasmid vectors can be directly introduced into the host cell, or fragments of the gene of interest comprising the insertion elements can be obtained by enzyme digestion of the plasmid vector, and the gene fragments can be further integrated into the genome of the host cell.

The fourth aspect of the present application provides a recombinant strain comprising the polynucleotide molecule provided in the second aspect of the present application, or the nucleic acid construct provided in the third aspect of the present application.

In some embodiments, the polynucleotide molecule is integrated into the genome of the host cell; preferably, the host cell is a eukaryotic cell; more preferably is *Saccharomyces cerevisiae.*

In some embodiments, a nucleic acid construct comprising a nucleotide sequence encoding the nerolidol synthase may be directly included in the recombinant strain, e.g., the nucleic acid construct is present alone in a host cell in the form of a plasmid, expressing the nerolidol synthase.

In other embodiments, the polynucleotide molecule is integrated into the genome of the host cell. The polynucleotide molecule is integrated into the genome of the host cell, which is beneficial to the long-term stable expression of the target gene, so as to obtain recombinant strain capable of stable inheritance.

A person skilled in the art can integrate polynucleotide molecules into the genome of the host cell by conventional methods, and is not limited herein by the present application. For example, the target gene can be connected between two insertion elements, and the target gene can be inserted into the genome of the host cell through the insertion element. For example, the insertion element can be the left and right homologous arms of Leu2, the left and right homologous arms of Ura3, the left and right homologous arms of YPRCdelta15, and the homologous arms of different genes can integrate the gene of interest into different locations in the host cell genome. The inventors have found that the recombinant strain of the present application can be obtained by inserting the target gene into each site that does not interfere with the normal physiological metabolism of the host cell.

In some embodiments, the copy number of the polynucleotide molecule is 1-3 in the genome of the recombinant strain.

In some embodiments, the recombinant strain is capable of endogenously and/or exogenously expressing at least one selected from acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase.

In some embodiments, the copy numbers of the acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase in the genome of the recombinant strain are 2, 2, 4, 2, 2, 2, 2, and 2, respectively.

The inventors have found that *Saccharomyces cerevisiae* can endogenously synthesize FPP, and therefore in some preferred embodiments, the use of *Saccharomyces cerevisiae* as host cell facilitates obtaining recombinant strains that efficiently synthesize nerolidol.

In some embodiments, the recombinant strain further comprises knockout or downregulation of at least one of the genes encoding FPP hydrolase DPP1, FPP hydrolase LPP1, citrate synthase, malate synthase, or squalene synthase. The inventors have found that FPP hydrolase DPP1 (diacylglycerol pyrophosphate phosphatase 1) and FPP hydrolase LPP1 (lipid phosphate phosphatase 1) have the ability to hydrolyze FPP to produce farnesol, and citrate synthase (CIT2) and malate synthase (MLS1) can consume acetyl coenzyme A; squalene synthase (ERG9) synthesizes squalene with FPP as substrate, and these enzymes competitively consume substrates required for nerolidol biosynthesis. Therefore, knockout or downregulation of the encoding genes of these enzymes reduces the expression of these enzymes in recombinant strains, which is beneficial to improving the efficient synthesis of nerolidol in recombinant strains.

The downregulation as described in the present application has its general meaning, and can be understood as inhibition of gene expression in the present application, resulting in a decreased amount of expression of the protein regulated by the gene.

The knockout as described in the present application has its general meaning and refers to inactivation or deletion of a specific gene through a certain pathway, thereby the protein encoded by which is reduced in expressing or not expressing.

The fifth aspect of the present application provides the use of nerolidol synthase in the first aspect of the present application, the polynucleotide molecule in the second aspect of the present application, the nucleic acid construct in the third aspect of the present application or the recombinant strain in the fourth aspect of the present application in the production of nerolidol.

The sixth aspect of the present application provides a method for preparing nerolidol, comprising biosynthesizing nerolidol using the recombinant strain in the fourth aspect of the present application.

In some embodiments, the recombinant strain is capable of endogenously and/or exogenously expressing at least one selected from acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase.

The inventors have found that introducing the target gene into a host cell capable of endogenously expressing at least one of enzymes selected from the enzymes in the mevalonate pathway and farnesene pyrophosphate synthase facilitates further improvement of nerolidol production.

In some embodiments, a nucleotide sequence which encodes at least one selected from acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase can be further linked into the nucleic acid construct, so that the recombinant strain can efficiently synthesize FPP, thereby contributing to further improvement of the yield of nerolidol.

In some embodiments, the copy numbers of acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase in the genome of the recombinant strain are 2, 2, 4, 2, 2, 2, 2, and 2, respectively.

In some embodiments, further comprises knockout or downregulation of at least one of the genes encoding FPP hydrolase DPP1, FPP hydrolase LPP1, citrate synthase, malate synthase, or squalene synthase.

In some embodiments, when the nucleic acid construct used comprises an inducible promoter, it may further include knockout of transcription inhibitor of the recombinant strain, for example, when GAL (galactose-inducible) promoter is used, transcription inhibitor GAL80 may be knocked out, and knockout of transcription inhibitor enable the recombinant strain to autonomously express the target gene without the need of an inducer, and reduces fermentation cost.

The nerolidol synthase and use thereof of the present application will be described by specific examples below. The following examples are used to further illustrate the present disclosure, but should not be construed as limiting the scope of the present disclosure. The plasmids involved in the following examples are all well-known to those skilled in the art. If specific techniques or conditions are not indicated in the examples, the test is carried out according to the techniques or conditions described in the literature in the art or according to the product specification. Reagents or instruments used without indicating the manufacturer are commercially available conventional products.

### Example 1 Functional identification of nerolidol synthase in Tanacetum cinerariifolium

### 1.1 Screening of potential nerolidol synthetic genes from Tanacetum cinerariifolium

RNA was extracted from samples taken from different tissues of *Tanacetum cinerariifolium* at different stages, and the second and third generation transcriptome sequencing was carried out; *Tanacetum cinerariifolium* transcriptome protein sequences comprising both Pfam domains PF01397 and PF03936 of terpene synthase were searched from the second and third generation transcriptome data, and 166 potential protein sequences were obtained. CD-Hit was used to conduct clustering to the found protein sequences to eliminate redundancy, and sequences with more than 90% sequence similarity were defined as a same class, results in a total of 33 classes were obtained; in each class, according to sequence integrity, protein sequences with a length greater than 500 were selected as candidate genes, and the genes with the highest expression level were further selected as test genes, a total of 24 genes to be verified were obtained, and the genes were named from CCJ-TPS01 to CCJ-TPS24.

### 1.2 Identification of nerolidol synthase and its gene in Tanacetum cinerariifolium

### 1.2.1 Construction of yeast expression universal vector

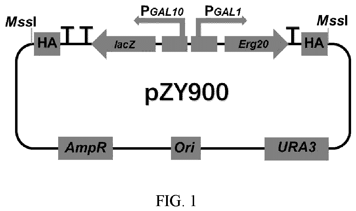

Specific construction process of plasmid pZY900: using the genome of *Saccharomyces cerevisiae* S288c (extraction method see: Xiaowei Li. Engineering Acetyl-CoA Pathway to Construct Efficient Biosynthesis Platform of Saccharomyces Cerevisiae [D]. Wuhan University, 2015. 2.3.6 Yeast genome DNA extraction method) as the template, amplify with primers 900-1F/1R, 900-2F/2R, 900-6F/6R and 900-7F/7R to obtain fragments 9001 (left homologous arm of Leu2), 9002 (terminator tTDH2), 9006 (gene ERG20 and terminator tERG20), 9007 (right homologous arm of Leu2) respectively; using the genome of *Saccharomyces cerevisiae* CEN.PK2-1D (extraction method see: Xiaowei Li. Engineering Acetyl-CoA Pathway to Construct Efficient Biosynthesis Platform of Saccharomyces Cerevisiae [D]. Wuhan University, 2015. 2.3.6 Yeast genome DNA extraction method) as the template, amplify with primers 900-3F/3R and 900-5F/5R to obtain fragments 9003 (terminator tCYC1) and 9005 (promoters pGAL1 and Pgal10) respectively; using pCAS (see literature Zhang, Yueping et al."A gRNA-tRNA array for CRISPR-Cas9 based rapid multiplexed genome editing in *Saccharomyces cerevisiae.*" Nature communications vol. 10,1 1053. 5 Mar. 2019, doi:10.1038/s41467-019-09005-3) as the template, amplify with primers 900-4F/4R to obtain fragments 9004 (nonsense gene lac Z, used for replacement of target gene); using pRS426 as the template, amplify with primers 900-8F/8R, 900-9F/9R, 900-10F/10R to obtain plasmid backbone (MssI restriction site, and screening markers (AmpR, URA3, etc.) introduced). pZY900 was constructed by DNA assembly (also known as Yeast assembly, Xiaowei Li. Engineering Acetyl-CoA Pathway to Construct Efficient Biosynthesis Platform of Saccharomyces Cerevisiae [D]. Wuhan University) in *Saccharomyces cerevisiae,* and then amplified in *Escherichia coli.* pZY900 was obtained after verified by enzyme digestion and sequencing. A schematic diagram of the construction of plasmid pZY900 is shown in FIG. 1, in which fragments 9001 (H A), 9002 (T), 9003 (T), 9004, 9005, 9006 and 9007 (HA) are sequentially ligated from left to right, and the rest is derived from plasmid backbone of pRS426.

The sequences of the primers used to construct plasmid pZY900 are shown in Table 1 below:

**Table 1**

| primer | Sequence (5'-3') |
|---|---|
| 900-1F | actaaagggaacaaaagctggagctctagtagtttaaacataacgagaacacacagggg (SEQ ID NO. 13) |
| 900-1R | cattaaagtaacttaaggagttaaatttaagcaaggattttcttaacttcttc (SEQ ID NO. 14) |
| 900-2F | gaagttaagaaaatccttgcttaaatttaactccttaagttactttaatgatttag (SEQ ID NO. 15) |
| 900-2R | tcgaaggctttaatttgcgcgaaaagccaattagtgtgata (SEQ ID NO. 16) |
| 900-3F | tagtatcacactaattggcttttcgcgcaaattaaagccttcgagc (SEQ ID NO. 17) |
| 900-3R | gggacgcgccctgtagcggctgaggtctcaacaggccccttttcctttg (SEQ ID NO. 18) |
| 900-4F | catgatatcgacaaaggaaaaggggcctgttgagacctcagccgctacagggcgc (SEQ ID NO. 19) |
| 900-4R | gaatttttgaaaattcaatataaatgtgagaccaccatgattacgccaagcg (SEQ ID NO. 20) |
| 900-5F | taatcatggtggtctcacatttatattgaattttcaaaaattcttactttttttttg (SEQ ID NO. 21) |
| 900-5R | atctctctctcctaatttctttttctgaagccattatagttttttctccttgacgttaaagt (SEQ ID NO. 22) |
| 900-6F | ttaacgtcaaggagaaaaaactataatggcttcagaaaaagaaattagga (SEQ ID NO. 23) |
| 900-6R | atgtacaaatatcataaaaaaagagaatctttttaaaaaaaatccttggactagtcacg (SEQ ID NO. 24) |
| 900-7F | actagtccaaggattttttttaaaaagattctctttttttatgatatttgtacataaac (SEQ ID NO. 25) |
| 900-7R | gcgccattcgccattcaggctgcgcaactgttgtttaaacgacaacgaccaagctcaca (SEQ ID NO. 26) |
| 900-8F | gatgtgagcttggtcgttgtcgtttaaacaacagttgcgcagcctgaatg (SEQ ID NO. 27) |
| 900-8R | tcaacagtatagaaccgtggatgatgtggtttctacaggatctgacattattattgttg (SEQ ID NO. 28) |
| 900-9F | atagtcctcttccaacaataataatgtcagatcctgtagaaaccacatcatccacggtt (SEQ ID NO. 29) |
| 900-9R | agggcttaccatctggccccagtgctgcaatgataccgcgcgacccacgctcaccggct (SEQ ID NO. 30) |
| 900-10F | tgataaatctggagccggtgagcgtgggtcgcgcggtatcattgcagcactggggccag (SEQ ID NO. 31) |
| 900-10R | cgatagcgcccctgtgtgttctcgttatgtttaaactactagagctccagcttttgttc (SEQ ID NO. 32) |

### 1.2.2 Construction of yeast expression vector

Primers were designed to connect the gene to be verified onto a universal vector. Here we demonstrate the validation process of CCJ_TPS23, the gene in *Tanacetum cinerariifolium* that was finally validated to have nerolidol synthase activity.

CCJ_TPS23-F/R was designed as a specific gene primer pair. cDNA of *Tanacetum cinerariifolium* was (RNA was extracted from *Tanacetum cinerariifolium* ovule tissue by RNAprep Pure Plant Plus Kit (Cat No. DP441) of TIANGEN Company, cDNA was obtained by reverse transcription of RNA by HiScript II 1st Strand cDNA Synthesis Kit (+gDNA wiper) (Cat. No. R212) of Vazyme) used as the template, and CCJ_TPS23 gene fragment was amplified by PCR using Phanta high fidelity enzyme of Vazyme Company. After gel recovery was carried out by using Tiangen gel recovery kit, it was ligated into BsaI cleaved yeast expression vector pZY900 by methods for homologous recombination using homologous recombination kit of Yeasen Company. After sequencing confirmation, the yeast expression vector comprising this gene was obtained and named pYR013. The plasmid construction schematic diagram is shown in pYR013 in FIG. 2, in which the lac Z gene in pZY900 is replaced by CCJ_TPS23 gene.

CCJ_TPS23-F/R primer sequences are shown in Table 2 below:

**Table 2**

| primer | Sequence (5'-3') |
|---|---|
| CCJ_TPS23-F | acaaaggaaaaggggcctgtttaaaacatggagtttatatattccttgatg (SEQ ID NO. 33) |
| CCJ_TPS23-R | tttttgaaaattcaatataaatgatcatcaacaattgtgttacac (SEQ ID NO. 34) |

### 1.2.3 Strain construction

Plasmid pYR013 was introduced by lithium acetate method (Xiaowei Li. Engineering Acetyl-CoA Pathway to Construct Efficient Biosynthesis Platform of Saccharomyces Cerevisiae [D]. Wuhan University, 2015. 2.3.14 *Saccharomyces cerevisiae* LiAc transformation) into strain JCR27 (for the construction of yeast strain JCR27 see literature Siemon, Thomas et al."Semisynthesis of Plant-Derived Englerin A Enabled by Microbe Engineering of Guaia-6,10(14)-diene as Building Block." Journal of the American Chemical Society vol. 142,6 (2020): 2760-2765. doi: 10.1021/jacs.9b12940) and the engineered strain was named CCJ-1.

Plasmid pZY900 was introduced into strain JCR27 using the same method as a control strain, and was named S900.

### 1.2.4 Strain fermentation and product identification

CCJ-1 and S900 were seeded respectively into SC-URA liquid medium (Xiaowei Li. Engineering Acetyl-CoA Pathway to Construct Efficient Biosynthesis Platform of Saccharomyces Cerevisiae [D]. Wuhan University, 2015, uracil-deficient medium), and incubated under 30°C, 200 rpm with shaking overnight; transferred with an initial OD600=0.1 into 45 ml of YPDHG liquid medium (20 g/L tryptone, 10 g/L yeast extract, 10 g/L glucose, 10 g/L galactose) on the next day, followed by incubation for 72 hours under 30°C, 200 rpm shaking, with additional 5 ml isopropyl myristate. The oil layer was collected and diluted with n-hexane to a suitable concentration, and the product was detected using GC-MS.

TRACE GC ULTRA gas chromatography of Thermo Fisher Scientific equipped with AS 3000 autosampler, split/splitless injector, and TSQ QUANTUM XLS MS equipped with triple quadrupole detector.

TR-5MS column (30m× 0.25 mm ×0.25 um) was used as the chromatographic column. The carrier gas is high purity helium at a flow rate of 1 mL/min. Acetone was used for needle wash. Injection volume is 1 uL, split ratio is 50. The temperature of injection port is 240°C, and the temperature of ion transport tube is 270°C.

Detection procedure: Initial column temperature is 50°C, hold for 1 min; raise temperature to 280°C at 15°C/min, hold for 1 min; raise temperature to 300°C at 20°C/min, hold for 2 min.

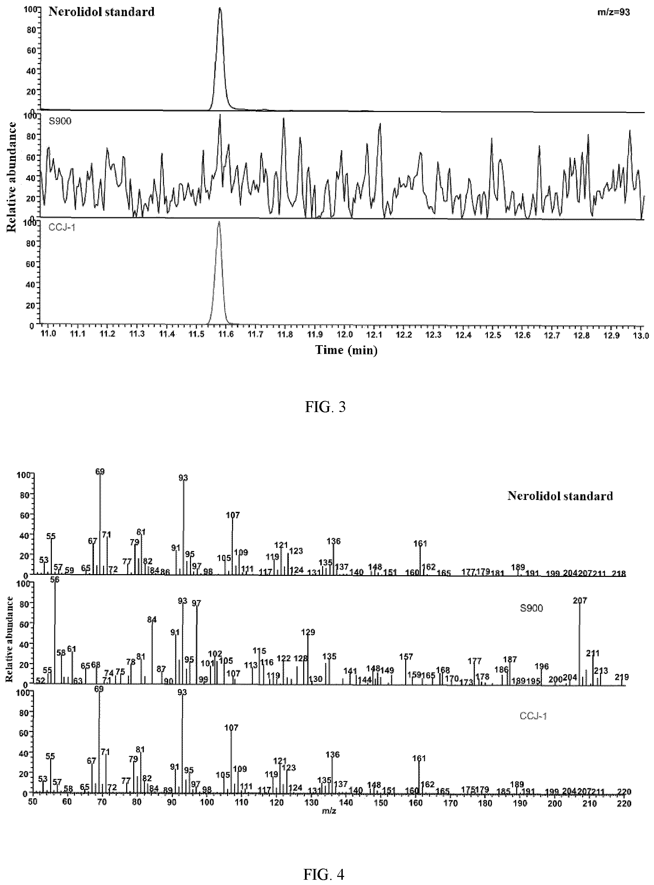

The extracted ion flow chromatogram of nerolidol characteristic ion m/z=93 in CCJ-1 and S900 fermentation products is shown in FIG. 3, and the mass spectrum of nerolidol is shown in FIG. 4. By comparing the chromatogram retention time and mass spectrum fragments with nerolidol standard, it can be determined that nerolidol can be synthesized in strain CCJ-1, but not in strain S900. These results indicate that the protein CCJ_TPS23 gene encodes is a nerolidol synthase.

Plasmid pYR013 was digested with MssI (see the instruction manual of MssI enzyme for conditions of enzyme digestion system) to obtain linearized fragments, which were integrated into strain JCR27 by lithium acetate method to obtain strain CCJ-2. The strain was seeded into YPD liquid medium and incubated under 30°C, 200 rpm with shaking overnight; transferred with an initial OD600=0.1 into 45 ml of YPDHG liquid medium on the next day, followed by incubation for 72 hours under 30°C, 200 rpm shaking, with additional 5 ml isopropyl myristate. The oil layer was collected and the product was detected using GC-MS, the shake flask fermentation yield of nerolidol of strain CCJ-2 was 271 mg/L.

### Example 2 Functional Identification of Nerolidol Synthetase in Astilbe chinensis

### 2.1 Screening of potential nerolidol synthetic genes from Astilbe chinensis

According to the same method, RNA was extracted from samples taken from different tissues of *Astilbe chinensis* at different stages, and the second and third generation transcriptome sequencing was carried out; *Astilbe chinensis* transcriptome protein sequences comprising both Pfam domains PF01397 and PF03936 of terpene synthase were searched from the second and third generation transcriptome data, and 89 potential protein sequences were obtained. CD-Hit was used to conduct clustering to the found protein sequences to eliminate redundancy, and sequences with more than 90% sequence similarity were defined as a same class, results in a total of 25 classes were obtained; in each class, according to sequence integrity, protein sequences with a length greater than 500 were selected as candidate genes, and the genes with the highest expression level were further selected as test genes, a total of 17 genes to be verified were obtained, and the genes were named from ACH-TPS01 to ACH-TPS17.

### 2.2 Identification of nerolidol synthase and its gene in Astilbe chinensis

### 2.2.1 Construction of yeast expression vector

Primers were designed to construct the gene to be verified onto a universal vector. Here we demonstrate the validation process of genes ACH_TPS07, ACH_TPS08, and ACH_TPS09 that were finally validated to have nerolidol synthase activity in *Astilbe chinensis.*

ACH_TPS07-F/R, ACH_TPS08-F/R, and ACH_TPS09-F/R were designed as specific gene primer pairs. cDNA of *Astilbe chinensis* was (RNA was extracted from *Astilbe chinensis* ovule tissue by RNAprep Pure Plant Plus Kit (Cat No. DP441) of TIANGEN Company, cDNA was obtained by reverse transcription of RNA by HiScript II 1st Strand cDNA Synthesis Kit (+gDNA wiper) (Cat. No. R212) of Vazyme) used as the template, and ACH_TPS07, ACH_TPS08, and ACH_TPS09 gene fragments were amplified by PCR using Phanta high fidelity enzyme of Vazyme Company. After gel recovery was carried out by using Tiangen gel recovery kit, it was ligated into BsaI cleaved yeast expression vector pZY900 by methods for homologous recombination using homologous recombination kit of Yeasen Company. After sequencing confirmation, the yeast expression vector comprising this gene was obtained and named pYR013. The plasmid construction schematic diagram of plasmids pYR007, pYR006 and pYR010 are shown in FIG. 2 as pYR007, pYR006 and pYR010, in which the lac Z gene in pZY900 is replaced by genes ACH_TPS07, ACH_TPS08 and ACH_TPS09, respectively.

Primer sequences are shown in Table 3 below.

**Table 3**

| Plasmid | Primer | Sequence (5'-3') |
|---|---|---|
| pYR007 | ACH_TPS07-F | acaaaggaaaaggggcctgtttaaaacaaacattgtatatgctcctcaag (SEQ ID NO. 35) |
| | ACH_TPS07-R | tttttgaaaattcaatataaatggcaccccctccttcc (SEQ ID NO. 36) |
| pYR006 | ACH_TPS08-F | acaaaggaaaaggggcctgtctatgtaacgtttatcatatttaaaacaaacattg (SEQ ID NO. 37) |
| | ACH_TPS08-R | tttttgaaaattcaatataaatggcaccccctccttcc (SEQ ID NO. 38) |
| pYR010 | ACH_TPS09-F | acaaaggaaaaggggcctgtctatgtaacgtttatcatatttaaaacaaacattg (SEQ ID NO. 39) |
| | ACH_TPS09-R | tttttgaaaattcaatataaatggcaccccctccttcc (SEQ ID NO.40) |

### 2.2.2 Strain construction

According to the same method in 1.2.3 of Example 1, plasmidpYR007 was introduced into strain JCR27 and the engineered strain was named LXF-1; plasmid pYR006 was introduced into strain JCR27 and the engineered strain was named LXF-1-1; plasmid pYR010 was introduced into strain JCR27 and the engineered strain was named LXF-1-2. Plasmid pZY900 was introduced into strain JCR27 as a control strain, and was named S900.

### 2.2.3 Strain fermentation and product identification

Strains LXF-1, LXF-1-1, LXF-1-2 and S900 were seeded respectively into SC-URA liquid medium, and incubated under 30°C, 200 rpm with shaking overnight; transferred with an initial OD600=0.1 into 45 ml of YPDHG liquid medium on the next day, followed by incubation for 72 hours under 30°C, 200 rpm shaking, with additional 5 ml isopropyl myristate. The oil layer was collected and the product was detected using GC-MS, under the same conditions as in 1.2.4 of Example 1.

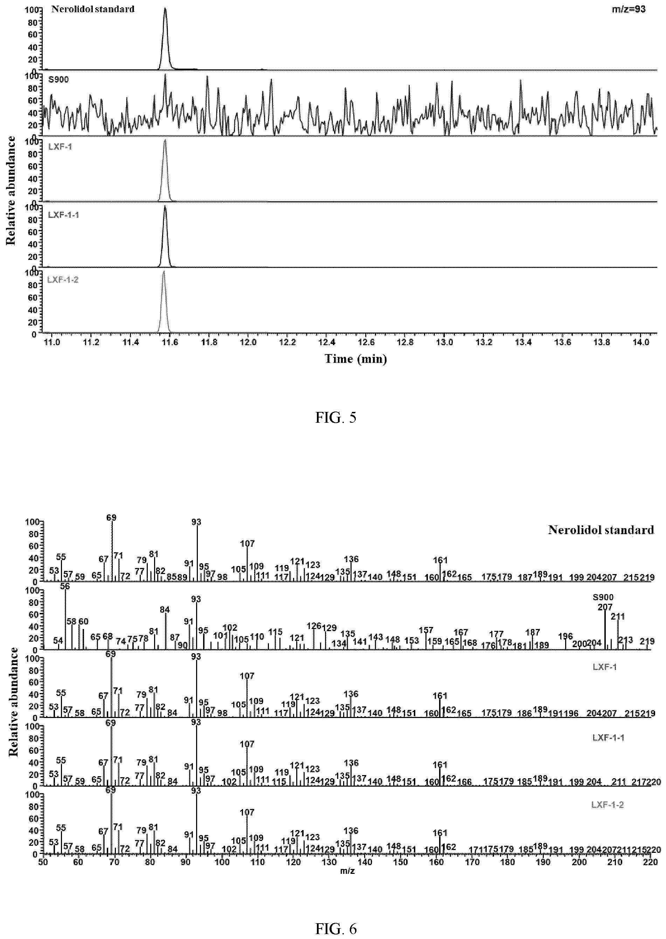

The extracted ion flow chromatogram of nerolidol characteristic ion m/z=93 in LXF-1, LXF-1-1, LXF-1-2 and S900 fermentation products is shown in FIG. 5, and the mass spectrum of nerolidol is shown in FIG. 6. By comparing the chromatogram retention time and mass spectrum fragments with nerolidol standard, it can be determined that nerolidol can be synthesized in strain LXF-1, LXF-1-1, LXF-1-2, but not in strain S900. These results indicate that the proteins ACH_TPS07, ACH_TPS08 and ACH_TPS09 gene encodes are nerolidol synthases.

Plasmid pYR007, pYR006 and pYR010 were digested respectively with MssI (see the instruction manual of MssI enzyme for conditions of enzyme digestion system) to obtain linearized fragments, which were integrated into strain JCR27 by lithium acetate method to obtain strains LXF-2, LXF-2-6 and LXF-2-10. The strains were seeded into YPD liquid medium and incubated under 30°C, 200 rpm with shaking overnight; transferred with a initial OD600=0.1 into 45 ml of YPDHG liquid medium on the next day, followed by incubation for 72 hours under 30°C, 200 rpm shaking, with additional 5 ml isopropyl myristate. The oil layer was collected and the product was detected using GC-MS, the shake flask fermentation yields of nerolidol of strains LXF-2, LXF-2-6 and LXF-2-10 were 269 mg/L, 240mg/L and 221mg/L respectively.

### Example 3 Functional Identification of Nerolidol Synthetase Artemisia argyi

### 3.1 Screening of potential nerolidol synthetic genes from Artemisia argyi

*Artemisia argyi* transcriptome protein sequences containing both Pfam domains PF01397 and PF03936 of terpene synthase were searched from *Artemisia argyi* transcriptome data (https://www.ncbi.nlm.nih.gov/bioproject/PRJNA722539), and 167 potential protein sequences were obtained. CD-Hit was used to conduct clustering to the found protein sequences to eliminate redundancy, and sequences with more than 90% sequence similarity were defined as a same class, results in a total of 47 classes were obtained; in each class, according to sequence integrity, protein sequences with a length greater than 500 were selected as candidate genes, and the genes with the highest expression level were further selected as test genes, a total of 29 genes to be verified were obtained, and the genes were named from Arar-TPS01 to Arar-TPS29.

### 3.2 Identification of nerolidol synthase and its gene in Artemisia argyi

### 3.2.1 Construction of yeast expression vector

Primers were designed to construct the gene to be verified onto a universal vector. Here we demonstrate the validation process of genes Arar-TPS27 and Arar-TPS28 that were finally validated to have nerolidol synthase activity in *Artemisia argyi.* The genes Arar-TPS27 and Arar-TPS28 derived from *Artemisia argyi* were directly synthesized according to the codon optimization of *Saccharomyces cerevisiae,* and the sequences are shown in SEQ ID NO.7 and SEQ ID NO.8.

Arar-TPS 27-F/R and Arar-TPS 28-F/R were designed as specific gene primer pairs, gene fragments obtained by PCR amplification was used as the template, and Arar-TPS27 and Arar-TPS28 gene fragments were amplified by PCR using Phanta high fidelity enzyme of Vazyme Company. After gel recovery was carried out by using Tiangen gel recovery kit, it was ligated into BsaI cleaved yeast expression vector pZY900 by methods for homologous recombination using homologous recombination kit of Yeasen Company. After sequencing confirmation, the yeast expression vector comprising this gene was obtained and named pArar-TPS27 and pArar-TPS28. The plasmid construction schematic diagrams of plasmids pArar-TPS27 and pArar-TPS28 are shown in FIG.2 as pArar-TPS27 and pArar-TPS28, in which the lac Z gene in pZY900 is replaced by genes pArar-TPS27 and pArar-TPS28 respectively.

Primer sequences are shown in Table 4 below.

**Table 4**

| Plasmid | Primer | Sequence (5'-3') |
|---|---|---|
| pArar-TPS27 | Arar-TPS27-F | ggaaaaggggcctgtttacttatcgtcgtcatccttgtaatcgaacatggagttaatgtattcttc (SEQ ID NO.41) |
| | Arar-TPS27-R | gaatttttgaaaattcaatataaatgacaatctctgttactag (SEQ ID NO.42) |
| pArar-TPS28 | Arar-TPS28-F | ggaaaaggggcctgtttacttatcgtcgtcatccttgtaatccatatcaatagccttgaagaacaag (SEQ ID NO.43) |
| | Arar-TPS28-R | gaatttttgaaaattcaatataaatgtctatcaacatcttgcatg (SEQ ID NO.44) |

### 3.2.2 Strain construction

According to the same method in 1.2.3 of Example 1, plasmid pArar-TPS27 was introduced into strain JCR27, and the engineered strain was named AH-1; plasmid pArar-TPS28 was introduced into strain JCR27, and the engineered strain was named AH-2; plasmid pZY900 was introduced into strain JCR27 as a control strain, and was named S900.

### 3.2.3 Strain fermentation and product identification

Strains AH-1, AH-2, and S900 were seeded respectively into SC-URA liquid medium, and incubated under 30°C, 200 rpm with shaking overnight; transferred with an initial OD600=0.1 into 45 ml of YPDHG liquid medium on the next day, followed by incubation for 72 hours under 30°C, 200 rpm shaking, with additional 5 ml isopropyl myristate. The oil layer was collected and the product was detected using GC-MS, under the same conditions as in 1.2.4 of Example 1.

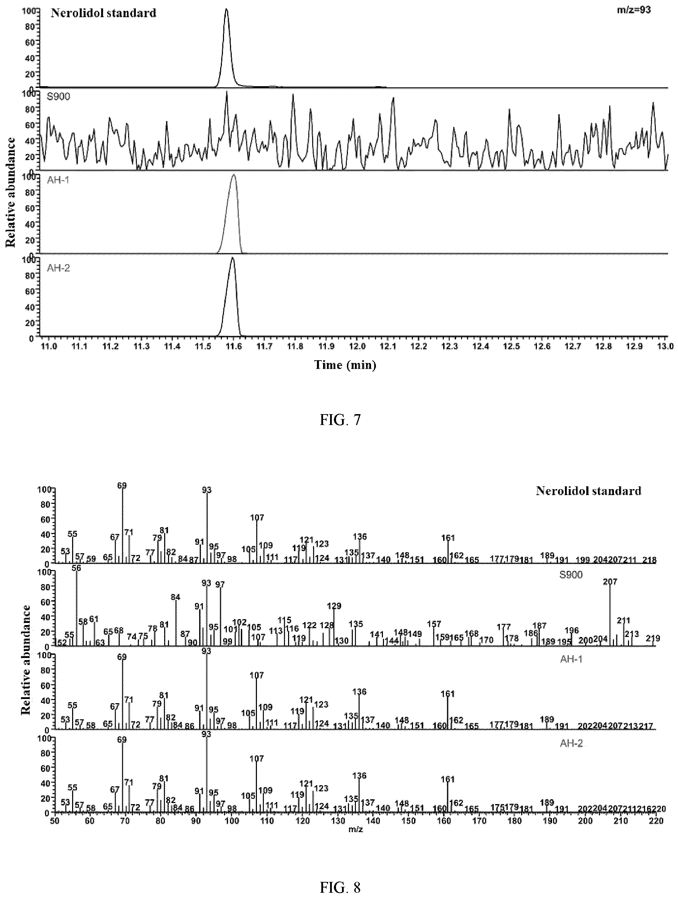

The extracted ion flow chromatogram of nerolidol characteristic ion m/z=93 in AH-1, AH-2 and S900 fermentation products is shown in FIG. 7, and the mass spectrum of nerolidol is shown in FIG. 8. By comparing the chromatogram retention time and mass spectrum fragments with nerolidol standard, it can be determined that nerolidol can be synthesized in strain AH-1 and AH-2, but not in strain S900. These results indicate that the proteins Arar-TPS27 and Arar-TPS28 gene encode are nerolidol synthases.

Plasmid pArar-TPS27 and pArar-TPS28 were digested respectively with MssI (see the instruction manual of MssI enzyme for conditions of enzyme digestion system) to obtain linearized fragments, which were integrated into strain JCR27 by lithium acetate method to obtain strains AH-27 and AH-28. The strains were seeded into YPD liquid medium and incubated under 30°C, 200 rpm with shaking overnight; transferred with an initial OD600=0.1 into 45 ml ofYPDHG liquid medium on the next day, followed by incubation for 72 hours under 30°C, 200 rpm shaking, with additional 5 ml isopropyl myristate. The oil layer was collected and the product was detected using GC-MS, the shake flask fermentation yields of nerolidol of strains AH-27 and AH-28 were 162 mg/L and 158 mg/L respectively.

### Comparative Example 1 Expression of an Existing Nerolidol Synthase Gene

Construction of plasmid comprising nerolidol synthase gene from *Celastrus angulatus* (CaNES 2), *Celastrus angulatus* (CaNES 1), *Tripterygium wilfordii* (TwNES), *Actinidia chinensis* (AcNES 1), *Fragaria x anassa* (FaNES 1), *Camellia sinensis* (CsNES2), and *Laggera pterodonta* (LpNES1)

The nerolidol synthase from different sources used in this comparative example are nerolidol synthases widely studied in the field and recognized as having high yield.

The nucleotide sequence of nerolidol synthase from *Celastrus angulatus* after *Saccharomyces cerevisiae* codon optimization is as follows (5'-3'), and the gene is named CaNES2.

ATGGACTTTTTTGATTGTTCCAGATCTTCCATTATTCCATTGAAGGCTTCCTCCCAAATT
GTTCCAGCTGATTCTACTAACAAGTGGGGTACTGTCGAATCTAACCACAACTCTGCTCCAACT
ACCCCATTAAACGACAGAATTTACGTTGAACACGCTCACATGGTCAAGGATTTCAAGAGAATC
ATGAACGTCGCTGGTGAAGACCCATCTGAAGGTTTGGCTATCATCGATGCTGTCCAAAGATTG
GTTGTCGACCATCATTTCCAAGAAGAAATTCACTCCATCCTACAAAAGCATAAGGAATTGGCT
ACCTCTACCACCACACACGGTGACTGTATGACCACCTCTTTGAGATTCAGATTGTTGAGACAA
GAAGGTTACTACGTCTCTGCTGATGTTTTCGAAGGTCTAAAAGATGAAGAAGGTAAGTTCGAC
CAAAACTTGAGTGGTGACATCAAGGGTTTGATGGCCTTGTACGAAGCTTCTCAATTCTCCATG
GAAGGTGAAAACATCTTGGATGAAGCCAGAGACTACTCTGCCCGTTTATTGAATGCTTGTGTT
ACCCAATTGGGTCACGATCAAGCTCGTATTGTCGAACACACTCTGACTCACCCACACCACAA
GTCTCTAGCCAGATTCATGGCCAAGAACTTCTTGAGAGATTTCCATTGCACTAACGGTTGGATT
GACGACTTGAAGAAGTTGGCCAAAGCTGACATCGACATGGCTCAATCCACTTTCCAAAACGA
AGTTGTTCAAATTTCTCAATGGTGGAAGGAATTAGGCTTAGCTGAAGAATTGAAATTTGCTCG
TGACCAACCAGTCAAATGGTACATCTGGACCATGACTTGTGACGAAGATCCATCTTTCTCCGA
ATTGAGAATCAACTTGACCAAGCCAATCTCTTTCGTCTACTTGATTGATGACATCTTCGATGTT
TATGGTACCTTGCAAGAAGTCACTGATTTCACCGAAGCTGTTCACAGATGGGACCACGACGCC
ATTGAACAATTGCCATACCACATGAAGATTTGTTTCAAGGCTTTGGACGATATCACCAACGAA
ATCTCCTACAAGGTCTACAAGCAATACGGATGGAACCCTTTAGACTCCCTCAGAAAGTCTTGG
GGTAGATTGTGTAACGCTTTCCTAACTGAAGCCAAGTGGTTCGCTTCTGGTCATTTGGCTAAG
GCTGAAGAATATTTAGAAAACGGTATCATTTCCTCTGGTGTTCCTGTTGTTTTGTTGCACTTCT
TTTTTCTTAGTGGTGAAGGTGTTACTCAAAAGTCCGTTGAAACCATTGACAACACTCCAGACA
TTGTCTCCGCTGCAGCCGCTATCTTGCGTTTATGGGATGATTTGGGTTCAGCTAAGGACGAAG
ACCAAGATGGTAAGGACGGTTCTTACTTGGCCTGTTACACTAATGAAAACCCAGGTTGTTCTT
TGGAAGATGCTGAGAAGCACGTTAAGTCCAAGATCTGTGATGAATGGAAATTGTTGAACAAG
GAGTGTTTGTCTCAAAAGAACCCATTCTCTGGTTCCTTCCACAGAGCTTGCTTGAATGTTGCT
AGAATGGTTCCATTGATGTACGAATACGACAAGAACAGAAGATTGCCAAGATTGGAAGAATTC
TTGAAGAGTATCTTGATCAAATGA (SEQ ID NO.99)

The nucleotide sequence of nerolidol synthase from *Celastrus angulatus* after *Saccharomyces cerevisiae* codon optimization is as follows (5'-3'), and the gene is named CaNES 1.

ATGAACTTGCCAATGACTCTAAGCTCCAAGTCCTTATTCACTTCTTCCAGAGCCTCTAT
CAGCTCTTCTTTCAAGACTATTTCCCAAATTGAAATTGCTAAAACTATCGCCAACTGTGTCCCA
TTGATTCCAGAACACCACAAGTGGTCTATTCACGATGTTTACCACAAATTAAGATTTTCCAACA
CTACAACCCCACCATTTAAGCAATTGAATCATTTGACCAAGCCATGCACCCCAAACGACCACA

TTTCTAAGGAACACGAAAGAAAGGTCAAGGCTTTCAAGAGGATTTTCAACATTGTTGGTGAA
GACCCAATCGAATCCTTGGTCATCATTGACGCTGTCCAACGTCTCGGTATTGACCATCACTTCC
AAGAAGAAATTAACACCATTCTTCAAGAATTGTACTACACTGAAGCTAACAACGCTCACATCG
GTGGTGGTGGTGGTTACTTCTCCCACTTGCACGAAGTTTCGCTAAGATTCAGATTGTTGCGTC
AACAAGGTTGTTACGTTTCTCCAGAAGTCTTTGACTGTTTCAAGGACAACAACGGTAAGTTC
AAAGAAAACTTGGGTGGTGACATCAACGGTTTGATGGCCTTATACGAAGCCTCCCATTTGTGT
TTGGAAGGTGAAAGTCTATTGCAAGAAGCTCGTGAATGTTCTGCCCAATTATTAAAGACCATC
GTCACTAACTTGAATCACTCTCAAGCTACCATGGTCGAACACACTTTGTCTCATCCACACCAC
AAGTCTTTCTCTAAATTCACCGGTAAGAAGTACTTGTCTCACGGTTTCCAAGATACCAACAAT
GGCTGGTTGAACATCTTGAAGGGTTTGGCTAAGATTGATTTCGACATGGCTCAATCCACTTAC
CAAAAGGAAATTGTCCAAATCTCTAAATGGTGGGAAGAATTGGGTTTGGCCAAGGAAATGTC
CTTCGCCAGAGATCAACCATTAAAGTGGTACATCTGGTCTATGGCTTGTATCACCGACCCATCC
TTATCTGAAGAAAGAATTGACTTGACTAAGTCCATCTCATTCATATACTTGATTGATGATATCTT
CGATTTGTACGGTACTCAAGACGAATTAACCTTGTTTACCCAAGTTGTTAACAGATGGGACTA
CGATGCTAGTGAACAATTGCCTCATTACATGAAGATTTGTTTCAAGGCTTTGGACGATATTATC
AACGAAATTTCTTTTAAGATCTACAAGAAGCACGGATGGAACCCAGTTGAATCTTTGAGAAA
GTCTTGGGGCAGACTATGCTCTGCATTCTTGGTTGAAGGTAGATGGTTCGCTTCAGGTTATGTC
CCAGCTTCCGAAGAATACTTGAAGAACGGTATCATCTCTTCTGGTGTTCATGTTGTCTGTGTTC
AATTGTTCTTCTTGTTGGGTAGACAAGATATAATTACTAAGCAATCTGTCGAAATCATTGACAA
CGATCCAGCCATCGTCTCCTCTACTGCTCAAATCTTGAGATTGTGGGATGACCTGGGTTCTGCT
AAGGATGAGGACCAAGATGGTCACGACGGTTCCTATTTAGACTACTATTTGAAAGAGCACCAA
GGTTGTTCCGTTAAGGATGCTGAAGAACACGTTATGTCTAAGATCTCTGACGCTTGGAAGCAA
TTGAACAAGGAATGTATGACGCCAACCCCATTCTCTCAATGTTTCATCAAGGCTACTTTGAACT
TGGCTAGAATGGTTCCTTTGATGTACTCTTACGACAACAACCAAAGATTACCATCTTTGGAAGA
TAACGTTAAATCTATCTTGTCCATTGCTTTGTGA (SEQ ID NO.100)

The nucleotide sequence of nerolidol synthase from *Tripterygium wilfordii* after *Saccharomyces cerevisiae* codon optimization is as follows (5'-3'), and the gene is named TwNES.

ATGGCTTTCTTCGGTTCCTCTAGATCTTCAATTATTCCACTAAAGACTATTAGTCAAATT
GTTACTGCTGACTCCACCAACAAGAGAGGTACCGTTGATTCTAACCACAAGTCCGCTCCAAC
CACTCCTTTGAACGACCGTATCTGCACCGAACACGCTCACAAGGTCAAGGATTTCAAGCAAA
TCATCAACATTGCTGGTGAAGATCCATCTGAAGGCTTGGCTATCATTGATGCTGTTCAAAGATT
GGGTGTTGACCATCACTTCCAAGATGAAATTCACACTATCTTGCAAAAGCATTACACCCTCGC
TACCGGTACGACCACCCATGGTGACTGTATGATCACTTCTTTGAGATTCAGATTACTTAGACAG
GAAGGTTACTACGTTTCTGCCGATGTTTTCGAAGGTTTAAAGGATGAAGAAGGTAAGTTCGAC
CAAAACTTGTCTGGTGACATCAAGGGTTTAATGGCTTTGTACGAAGCCTCCCAATTGTCCATG
GAAGGTGAAAACATCTTGGACGAAGCTAGAGACTACTCTTCCAGATTGTTGAACGAATGTGTT
ACCCAATTGGACCACGACCAAGCTAGAATCGTCGAACACACTTTGACCCACCCACACCACAA
ATCTCTAGCCCGTTTCATGGCTAAGAACTTCTTAAGAGACTTCCACGGTACTAACGGTTGGATT

GACGACTTGAAGAAGTTGGCCAAGGTTGACTTTGACATGGCCCAATCTACTTACCAAAAGGA
AGTCGTCCAAATCAGTCAATGGTGGAAGGAATTGGGTTTGGCTGAAGAATTGAAATTCGCTA
GAGATCAACCAGTCAAGTGGTACATCTGGACCACAACTTGTCACCAAGACCCATCTTTCTCGG
AATTGCGTATTAACTTAACCAAGCCAATTTCTTTCGTCTACTTGATTGATGATATCTTTGATGTCT
ACGGTACCCCACAAGAAGTTACTGACTTCACTGAAGCTGTCGATAGATGGGATCATGATGCCA
TCGACCAATTGCCATACTACATGAAGATCTGTTTCAAGGCTTTAGATGACATAACCAACGAAAT
CTCTTACAAGGTTTACAAGCAACACGGTTGGAACCCATTGGATTCATTGAGAAAATCTTGGGG
TAGATTATGTAACGCTTTCTTGACTGAAGCTCAATGGTTCGCTTCTGGTCACCTGCCAAAGGC
CGAAGAATACTTGGAAAATGGTATCATCAGCTCCGGTGTCCCAGTTGTTTTGCTGCATTTGTTC
TTTTTGTTGGGTGAAGGTGTCACTCAAAAATCCGTTGAAATGATTGACAACACTCCAGCTATT
GTCTCTGCTGCCGCTGCTATTTTGAGATTATGGGACGATTTGGGCTCTGCTAAGGACGAAGAC
CAAGATGGAAAGGATGGTTCTTATTTGGCATGTTACACTAATGAAAACCCAGGTTGTTCCTTG
GAAGACGCTGAAAAGCACGTTAAGTCTAAGATTTGTGATGAATGGAAACAATTAAATAAAGA
ATGTTTGTCCCAAAAGAACCCATTCTCTCCATCCTTTTTGAAGGCTTGCTTGAACGTTTCCAGA
ATGGTTCCTTTGATGTACGAATATGACGAAAACAGACGTTTGCCAAGATTGGAAGAATTCTTG
AAATCCATCTTAATCAAGtaa (SEQ ID NO.101)

The nucleotide sequence of nerolidol synthase from *Actinidia chinensis* after *Saccharomyces cerevisiae* codon optimization is as follows (5'-3'), and the gene is named AcNES 1.

ATGGCCACTGCCGCTGGTCCAATTGCCACCAACAACTCCCCACAAAACTCTAACGCTT
ACAGAACCCCAATCGCTCCATCTGTTCCAATCACCCACAAGTGGTCTATTGCTGAAGACTTGA
CTTGTATCTCCAACCCATCCAAGCATAACAACCCTCAAACCGGTTACAGATCTTTTTCTGATGA
ATTATACGTTAAGTACGAAGAAAAGTTGGAAGATGTCAGAAAGGCTTTGAGAGAAGTTGAAG
AAAACCCATTGGAAGGTTTAGTTATGATTGACGCTTTGCAAAGATTGGGAATTGATTATCACTT
CAGAGGTGAAATCGGTGCTTTCTTGCAGAAGCAACAAATCATTTCTTCTACTCCAGATGGTTA
CCCAGAACACGGTTTATATGAAGTCTCCACTTTATTCAGATTCTTGAGACAAGAAGGTCACAA
CGTTACTGCTGATGTTTTCAACAACTTCAAGGACAAGGAAGGTCGTTTCCGTTCAGAATTGTC
TACCGACATCAGAGGTTTGATGTCTTTGTACGAAGCTTCTCAATTAAGAATTGAAGGTGAAGA
CATCTTGGACCAAGCTGCTGACTTCTCCTCCCAATTGTTGGGTAGATGGACCAAGGATCCAAA
CCATCACGAAGCCAGATTGGTTTCTAACACTTTGACTCACCCATACCACAAGTCTTTGGCCAC
TTTCATGGGTCAAAAATTGTCCTATATGAACTGTAAGGGTCCAAACTGGGACGGCGTTGACAA
TTTGCAAGAACTCGCTAAGATGGATTTGACCATTGTTCAATCTATTCACCAAAAGGAAGTCTT
CCAAGTTAGTCAATGGTGGAAAGACACTGGTTTGGCTAATGAATTGAAGCTAGCCAGAAACC
AACCATTGAAGTGGTACATGTGGCCAATGGCTGCCTTGACCGACCCAAGATTTTCTGAAGAA
AGAGTTGAATTGACTAAGCCAATCTCTTTTATCTACATTATTGATGATATCTTCGACGTCTACGG
TACTATTGAAGAGTTGACTTTGTTCACCGATGCCGTCAACAGATGGGAATTGTCTGCTGTTGA
ACAATTGCCAGACTACATGAAGGTTTGTTTCAAAGCTTTGTACGACGTCACTAATGAAATTGC
TTACAAGATATACAAGAAGCACGGTCAAAACCCGATTGACTCCTTGCAAAAGACCTGGGCTT
CATTGTGTAACGCATTTCTGGTCGAAGCTAAATGGTTCGCTTCCGGTCATTTGCCAAATGCTGA

AGAATACTTGAAGAACGGTATCATCTCGTCTGGTGTCCACGTTGTTTTGGCTCACATGTTCTTC
TTATTAGGTGACGGTATCACTCAAGAATCCGTCGATTTGGTCGACGACTACCCAGGTATCTCCA
CCTCCATCGCTACCATCTTGAGATTATCCGACGATCTAGGTTCTGCCAAGGACGAAGACCAAG
ATGGTTACGATGGTTCCTACATCGAATGTTACATGAAAGAACATAAGGGTTCTTCTGTTGACTC
CGCTAGGGAAGAAGTTATCAGAATGATCTCTGAAGCTTGGAAGTGTTTGAACAAGGAATGCT
TGTCTCCAAACCCATTCTCTGAATCTTTCCGTATTGGTTCGTTAAACATGGCTAGAATGATTCC
AATGATGTACTCTTACGATGACAACCACAACCTTCCTATCTTGGAAGAACACATGAAGGCTAT
GATATACAACACCTCTCTAtaa (SEQ ID NO.102)

The nucleotide sequence of nerolidol synthase from *Fragaria x ananassa* after *Saccharomyces cerevisiae* codon optimization is as follows (5'-3'), and the gene is named FaNES1.

ATGAACGTCGAAACTAAGCACACTAGAACCATGGGTGACATTTTTGTCCAACACTCTC
AAAAGTTGGAACTGTTGAAGACAGTCTTGAGAAATGTTGCTGAATTGGACGCTCTTGAAGGT
TTGAACATGATTGATGCTGTCCAAAGATTAGGTATTGACTACAACTTCCAAAGAGAAATTGAC
GAAATCTTACACAAGCAAATGTCCATAGTTTCTGCCAGAGATGACTTGCACGAAGTCGCTTTG
AGATTCAGATTGTTGAGACAACACGGTTACTTCGTTCCAGAAGATGTTTTCAACAACTTCAAG
GACTCCAAGGGTACTTTCAAGCAAGTTTTGGGTGAAGACATCAAGGGCTTGATGTCTTTATAC
GAAGCTTCTCAATTGGGAACTGAAGGTGAAGACATTTTGGTGGAAGCTGAAAAGTTCTCTGG
TCACTTGTTAAAGACTTCTTTGTCTCACTTGGATCATCACAGAGTCAGAATTGTTGCTAACACT
TTGCGGAACCCACATCACAAGTCTTTGGCTCCATTCATGGCTCGTAACTTTTTCGTCACCTCCC
AAGCTACCAACTCTTGGCTAAACTTGTTGAAGGAAGTTGCCAAGACTGATTTCAACATGGTTA
GATCTCTCCACCAAAATGAAATTGTCCAAATGTCCAAGTGGTGGAAAGAATTGGGTTTAGCAA
AGGAATTAAAATTTGCTAGAGATCAACCATTAAAGTGGTACATCTGGTCTATGGCCTGTTTGAC
TGACCCAAAGTTGTCCGAAGAAAGAGTTGAATTGACCAAGCCAATCAGTTTCGTCTACTTGAT
TGACGATATCTTCGATGTCTACGGTACCTTGGATGACTTGATCTTGTTCACCGAAGCTGTTAAC
AGATGGGAAATCACTGCTATCGACCATCTGCCAGACTATATGAAGATCTGTTTCAAGGCTTTGT
ACGACATGACTAACGAATTCTCCTCCAAGGTTTACTTGAAGCATGGTTGGAACCCATTGCAAT
CTTTGAAGATTTCCTGGGCTAGCTTGTGCAATGCTTTCTTGGTTGAGGCCAAATGGTTCGCTTC
TGGTAAGCTCCCAAAGTCTGAAGAATATCTTAAGAACGGTATCGTCTCTTCCGGTGTCAACGT
TGTTTTAGTTCACATGTTCTTTTTACTAGGTCAAAACATCACCAGAAAGTCCGTTGAATTGTTG
AACGAAACCCCAGCTATTATTTCTTCTTCAGCTGCTATTTTGCGTTTGTGGGATGATTTGGGTT
CTGCTAAGGATGAAAACCAAGATGGTAATGACGGTTCCTACGTTAGATGTTACTTGGAAGAAC
ACGAAGGTTGTTCTATTGAAGAAGCCAGAGAAAAGACCATCAACATGATCTCTGACGAATGG
AAGAAGTTGAACCGTGAATTATTGTCTCCAAACCCTTTCCCAGCCTCCTTCACTTTGGCTTCAT
TGAACTTGGCTAGAATGATCCCTTTAATGTACTCTTACGATGGTAACCAATGTTTGCCATCCCTA
AAAGAATACATGAAATTGATGTTGTACGAAACCGTCTCCATGtaa (SEQ ID NO.103)

The nucleotide sequence of nerolidol synthase from *Camellia sinensis* after *Saccharomyces cerevisiae* codon optimization is as follows (5'-3'), and the gene is named CsNES2.

ATGGCCACCACCACTACAACTATCTCAACCACACACAAATGGTCTATCTCTGTTGCTGA
AGACTTGAAGTGTTTGACTCCTCATTGTACTTTCACCAACGAAATCTTAGCTAAGCAAGAAGA
ACAATTGGAAGAAGTCAGATCTGTCTTGAGAAAGGTTAGAGAAGAAGACTCTCTAAAGC
GTTTGGTTATGATCGACGCTTTGCAACGTCTGGGTATTGACTACCACTTCAGAGAAGACATCG
AAGCTGTTTTGCACTCCCAATACATGAAGTCCTCTTCCCACTCTTTCACTTACCAACACGAAG
ATTTGTACGAAGTTTCCACTAGATTCAGATTGTTGCGTCAACAAGGCCACAACGTTCCAACTG
ATGTTTTCGATAAGTTCAAGGACAAGGATGGTAGATTTAAGACCGAATTGTCTGAAGACATCA
GAGGTTTGATGGGTTTATACGAAGCCTCCCAGTTATCTATTGAAGGTGAAGATATTTTGGACCA
AGCTGCCGACTTCTCTGCTCAACAATTGAACGGTTGGTTGACCAGATTGGGTAACCATCACCA
AGCCAAGGTTATCTCTAACACTCTTGGTCATCCACAACATAAATCATTGGCTAAATTCATGGCT
AAGAATTTTTTGTCTGACTACAACGGTCCAAACGCCAATGTTTTGCAAGAACTAGCGAAGATG
GACTTCAACATGTCTCCATCCATGTACCAAAAGGAAATGTTGCAAGTCTCTAAGTGGTGGAAA
TCCACTGGTTTGGGTAACGAATTAAAGTACGCTAGAAACCAACCATTGAAATGGTACTTGTGG
TCCATGGCTACTTTAACCCACCCATCTTTGTCTGAACAAAGAGTTGAATTGACCAAGCCAATC
TCTTTGGTCTACATTATTGACGACATCTTCGACGTTTATGGTACCTTGGAACAATTAATTTTGTT
CACTGAAGCTGTTAACAGATGGGAATTGGCCGCTGTCAATGAATTGCCAGGTTACATGAAGAT
CTGTTTCAAGGTCTTGCAAGATAGTACTAATGAAATTGCTTACGGTGTCCACAAGAAGTACGG
TTGGAACCCAATTGATAGTTTGAGAAGAACCTGGGCTATCTTGTGTAACGCTTTCTTGGAAGA
AGCTAAGTGGTTCGCTTCTGGTCAATTGCCAAAGGCTGAAGAATACTTAAAGAACGGTATCGT
CTCCAGCGGTGTCCCAGCTGTCCTCGTCCACATGTTTTTCTTGTTGGGTCACGGTATGAACAA
GGAATCTGTCGATTTTACCATCGATAACCCAGACATTATCACTTCTACCGCCACCATTTTGAGAT
TATGGGACGATCTGGGTTCTGCAAAGGATGAAAACCAAGACGGTCACGATGGTTCTTACGTTG
ATTGCTATATGAAAGAAAACGAAGGTTCCACCGCTGAAACTGCTAGAAAGCAAGTTGTTGAC
ATGATCTCTGATGCTTGGAAGAGATTGAACCGTGAATGTTTGTCTCCAAACAGATTCTCCGCT
AACTTCACTCAAGGTTGTCTCAACACTGCCAGAATGGTTCCATTGATGTACTGTTACGATGAC
AACCACAACTTGCCTATTTTGGAGGAGCACATGAAGTCCATGTTCCACGACTCCGTCAGCTTA
TGA (SEQ ID NO.104)

The nucleotide sequence of nerolidol synthase from *Laggera pterodonta* after *Saccharomyces cerevisiae* codon optimization is as follows (5'-3'), and the gene is named LpNES1.

ATGAAGAAGGAATTATCCACACGTCACTCTGAAGCCTTGGAAGAAGTCAGAAACCTAT
TGAACAATGGTCCATTTAAGAACTTGCACATGGTTGACACTTTGCAAAAGTTGTGTATCGACC
ACCACTTCCAAGAAGAAATTGACATGATTTTCAAGATGTCCAACGTTCATGACTACCAAACCC
TATACGCCTTATCCTTATATTTCAGAATCTTTAGACAACAAGGTTACTACGTTCCAGCTGACGTT
TTCGCTAACTTTAAGCAAAAGGATGGTAGATTCAAAGGTGAAATTGCTCAAGATTTGAAGGGT
TTATTAGCTTTATACGAAGCTTCTCAATTATCTATTCCAGGTGAAGACATTTTGGAGGAAGCTG
CTGATTTCTCAAGATACTTGTTGGAACAAAAGATCCCATTTTTGGGTCAAGAAGAAGCCCTCA
AGGTTAACAACACCTTGGAACACTCCTACCAAAAGATCCCATCTAACTTCAGATTGAAGATCT
TCATCAAGCACTACTCTGGTACCACCATGTCTGACTTGGCTATGCTGGAATTGGCTAAGGCCA

AGTCTGTTCACGCTACTGAAGTTGAACAATTCTCCAGATGGTGGAAGGATCTAGGTTTGGCTC
AAGAATTGAAATTGGCCAGAAACCAACCATTGCACTGGTACTTGTGGCCAATGGCTGGTTTG
ACTGACTTGTCTTTGTCTGAAATCAGAATTGACTTGACCAAGCCAATCGCTTTGGTCTTCATTA
TTGATGACATCTTCGATGTCTACGGTACTTTGGACCAATTGATTTTATTCACTGAAGCTGTTACC
AGATGGGATTCCAATAACTTGGAACAATTGCCATACAATTTGAGAACTTGTGTTCAAGCTGTC
TTTGATGTTACTCACGAAATCTCTGATAAAATTTACAACAAGTACGGTTTCAACCCTATTGAAT
TCTTGAAGCAAACTTGGACTGATTTGTGTGAAGCTTTCTTAGTCGAAGCAAAGTGGTTCACCA
AAGGTTACATGCCAAAGGCTGAAGATTATTTAAAGAACGGTGCCGTCTCCACCGGTGCTCAC
GTTGTGATTGTCCACATGTTCTTCTTGTTGGGTGGTGCCACCTCCAAGGAAACTGCTCAAATC
ATTGACGAACACAAGGGTATCACTTCTTGCTTAGCCAAGATCTTGCGATTGTGGGACGATTTG
GGTTCTGCTAAGGACGTCAACCAAGACGGTCATGACGGCTCATACGTCACTTACTATATGAAT
GAAAACTCTGGATGTTCCATCCAAAACGCTCGTGAACATGTTATGAAGATGATCATCAACACC
TGGAAACAATTGAACAAAGAATGTTTGTCTCCAAACCCATTCTCTGCCACTTTCACCAAGGCT
TGTTTGAACTTGGCTAGAATGATTCCTTTGATGTACAACTACGATGAAAACCATAGTTTGCCAT
TGATCAAGGACTACATTAACTCCATGTTCtaa (SEQ ID NO.105)

Primers were designed to construct above nerolidol synthase genes onto universal vector pZY900. Primer sequences designed for each gene are shown in the table below. Using the synthesized genes above as template, according to the same method as described in 1.2.2 in Example 1, yeast expression vectors comprising CaNES2, CaNES1, TwNES, AcNES1, FaNES1, CsNES2 and LpNES1 genes were constructed and named pCaNES2, pCaNES1, pTwNES, pAcNES1, pFaNES1, pCsNES2 and pLpNES1, respectively. The plasmid construction schematic diagrams of plasmids pCaNES2, pCaNES1, pTwNES, pAcNES1, pFaNES1, pCsNES2 and pLpNES1 are shown in FIG.2, in which the lac Z gene in pZY900 is replaced by genes CaNES2, CaNES1, TwNES, AcNES1, FaNES1, CsNES2 and LpNES1, respectively.

| Plasmid | Primer | Sequence (5'-3') |
|---|---|---|
| pCaNES2 | CaNES2-1F | GATATCGACAAAGGAAAAGGGGCCTGTTCATTTGATCAAGAT ACTCTTCAAG (SEQ ID NO.106) |
| | CaNES2-1R | gaatttttgaaaattcaatataaATGGACTTTTTTGATTGTTCCAG (SEQ ID NO.107) |
| pCaNES1 | CaNES1-1F | GACAAAGGAAAAGGGGCCTGTTCACAAAGCAATGGACAAG ATAGAT (SEQ ID NO.108) |
| | CaNES1-1R | aagaatttttgaaaattcaatataaATGAACTTGCCAATGACTCTAAG (SEQ ID NO.109) |
| pTwNES | TwNES-1F | ATCGACAAAGGAAAAGGGGCCTGTttaCTTGATTAAGATGGAT TTCAAGAAT (SEQ ID NO.110) |
| | TwNES-1R | atttttgaaaattcaatataaATGGCTTTCTTCGGTTCCTCTAG (SEQ ID NO.111) |
| pAcNES1 | AcNES1-1F | CAAAGGAAAAGGGGCCTGTttaTAGAGAGGTGTTGTATATCAT AGCC (SEQ ID NO.112) |
| | AcNES1-1R | gaatttttgaaaattcaatataaATGGCCACTGCCGCTGGTC (SEQ ID NO.113) |
| pFaNES 1 | FaNES1-1F | ATCGACAAAGGAAAAGGGGCCTGTttaCATGGAGACGGTTTC GTACAAC (SEQ ID NO.114) |
| | FaNES1-1R | taagaatttttgaaaattcaatataaATGAACGTCGAAACTAAGCACACTAG (SEQ ID NO.115) |
| pCsNES2 | CsNES2-1F | GACAAAGGAAAAGGGGCCTGTTCATAAGCTGACGGAGTCGT GGAAC (SEQ ID NO.116) |
| | CsNES2-1R | aaagtaagaatttttgaaaattcaatataaATGGCCACCACCACTACAAC (SEQ ID NO.117) |
| pLpNES 1 | LpNES1-1F | CGACAAAGGAAAAGGGGCCTGTttaGAACATGGAGTTAATGT AGTCCT (SEQ ID NO.118) |
| | LpNES1-1R | gtaagaatttttgaaaattcaatataaATGAAGAAGGAATTATCCACACGTC (SEQ ID NO.119) |

Construction and shake-flask fermentation of strains comprising nerolidol synthase gene from *Celastrus angulatus* (CaNES2), *Celastrus angulatus* (CaNES1), *Tripterygium wilfordii* TwNES (KU588405), *Actinidia chinensis* (AcNES1), *Fragaria x anassa* (FaNES1), *Camellia sinensis* (CsNES2), and *Laggera pterodonta* (LpNES1)

Plasmid pCaNES2, pCaNES1, pTwNES, pAcNES1, pFaNES1, pCsNES2, and pLpNES1were linearized respectively with MssI, and fragments comprising the target genes were recovered using a gel recovery kit and introduced into strain JCR27 by lithium acetate method, and the engineered strains were named JCaNES2, JCaNES1, JTwNES, JAcNES1, JFaNES1, JCsNES2, and JLpNES1, respectively. The strains were respectively seeded into YPD liquid medium and incubated under 30°C, 200 rpm with shaking overnight; transferred with an initial OD600=0.1 into 45 ml of YPDHG liquid medium on the next day, followed by incubation for 72 hours under 30°C, 200 rpm shaking, with additional 5 ml isopropyl myristate. The oil layer was collected and the product was detected using GC-MS under the same detecting condition in 1.2.4 of Example 1, the shake flask fermentation yields of strains JCaNES2, JCaNES 1, JTwNES, JAcNES1, JFaNES1, JCsNES2 and JLpNES1 were 33.7 mg/L, 8.3 mg/L, 69 mg/L, 111 mg/L, 86.5 mg/L, 21 mg/Land 5.3 mg/L, respectively.

By comparing Examples 1, 2 and 3 with Comparative Example 1, it can be seen that the nerolidol synthase derived from *Tanacetum cinerariifolium, Astilbe chinensis* and *Artemisia argyi* of the present application has better performance than the existing nerolidol synthase.

### Example 4 Construction of A High-Yield Strain Comprising the Nerolidol Synthase CCJ_TPS23 Derived from Tanacetum cinerariifolium

### 4.1 Construction of high yield plasmid

Using the whole genome of *Saccharomyces cerevisiae* CEN.PK2-1D, plasmid pRS423, the whole genome of *Saccharomyces cerevisiae* CEN.PK2-1D, the whole genome of *Saccharomyces cerevisiae* S288C, the whole genome of *Saccharomyces cerevisiae* CEN.PK2-1D, *Tanacetum cinerariifolium* cDNA, the whole genome of CEN.PK2-1D, the whole genome of CEN.PK2-1D, and pRS426 plasmid as templates, amplify with primers 020-1F/R, 020-2F/R, 020-3F/R, 020-4F/R, 020-5F/R, 020-6F/R, 020-7F/R, 020-8F/R and 020-9F/R, respectively, to obtain the fragments which are left homologous arm (HA) of Ura3, histidine screening marker (HIS3), CYC1 terminator (T), tHMG1 gene, GAL1-GAL10 promoters (P_{GAL10} and P_{GAL1}), CCJ_TPS23, PGK1 terminator (T), right homologous arm (HA) of Ura3, and plasmid backbone. Then, the plasmid pYR020 was obtained by recombinant construction in *Saccharomyces cerevisiae* using the same yeast assembly method as in Example 1, and sequentially ligating the above fragments. The plasmid construction schematic diagram of plasmid pYR020 is shown in FIG. 9 as pYR020, which also includes the MssI restriction site, and the rest part derives from the pRS426 plasmid backbone.

Primer sequences are shown in Table 5 below.

**Table 5**

| Primer | Sequence (5'-3') |
|---|---|
| 020-1F | attaaccctcactaaagggaacaaaagcgtttaaacacgcagataattccaggtatttt (SEQ ID NO.45) |
| 020-1R | aatacgactcactatagggcgaattgggtaccttcgtttcctgcaggtttttgt (SEQ ID NO.46) |
| 020-2F | caaaaacctgcaggaaacgaaggtacccaattcgccctatagtgag (SEQ ID NO.47) |
| 020-2R | gttttgggacgctcgaaggctttaatttgctcacagcttgtctgtaagcg (SEQ ID NO.48) |
| 020-3F | ttgtctgctcccggcatccgcttacagacaagctgtgagcaaattaaagccttcgagcg (SEQ ID NO.49) |
| 020-3R | gtttgaaagatgggtccgtcacctgcattaaatcctaaacaggccccttttcctttgtc (SEQ ID NO.50) |
| 020-4F | taattacatgatatcgacaaaggaaaaggggcctgtttaggatttaatgcaggtgacgg (SEQ ID NO.51) |
| 020-4R | gaatttttgaaaattcaatataaatggttttaaccaataaaacagtcat (SEQ ID NO.52) |
| 020-5F | gttttattggttaaaaccatttatattgaattttcaaaaattcttactttttttttgg (SEQ ID NO.53) |
| 020-5R | taacacaattgttgatgatcattatagttttttctccttgacgttaaagt (SEQ ID NO.54) |
| 020-6F | gtcaaggagaaaaaactataatgatcatcaacaattgtgttacac (SEQ ID NO.55) |
| 020-6R | cgatttcaattcaattcaatttaaaacatggagtttatatattccttgatg (SEQ ID NO.56) |
| 020-7F | tatataaactccatgttttaaattgaattgaattgaaatcgatagatcaat (SEQ ID NO.57) |
| 020-7R | ttgaagctctaatttgtgagtttagtatacatgcatttacaacgaacgcagaattttcg (SEQ ID NO.58) |
| 020-8F | gtttaataactcgaaaattctgcgttcgttgtaaatgcatgtatactaaactcacaaat (SEQ ID NO.59) |
| 020-8R | gacggtcacagcttgtctgtgtttaaaccgtttaagggcaaatgtactct (SEQ ID NO.60) |
| 020-9F | agagtacatttgcccttaaacggtttaaacacagacaagctgtgaccgtc (SEQ ID NO.61) |
| 020-9R | tgcttcaaaatacctggaattatctgcgtgtttaaacgcttttgttccctttagtgagg (SEQ ID NO.62) |

Using the whole genome of *Saccharomyces cerevisiae* CEN.PK2-1D, plasmid pRS424, the whole genome of *Saccharomyces cerevisiae* S288C, the whole genome of *Saccharomyces cerevisiae* CEN.PK2-1D, *Tanacetum cinerariifolium* cDNA, the whole genome of *Saccharomyces cerevisiae* CEN.PK2-1D, the whole genome of *Saccharomyces cerevisiae* CEN.PK2-1D, and pRS426 plasmid as templates, amplify with primers 021-1F/R, 021-2F/R, 021-3F/R, 021-4F/R, 021-5F/R, 021-6F/R, 021-7F/R and 021-8F/R, respectively, to obtain the fragments which are left homologous arm (HA) of YPRCdelta15, tryptophan selection marker (TRP1), GPM1 terminator (T), GAL1-GAL10 promoter (P_{GAL10} and P_{GAL1}), CCJ_TPS23, PGK1 terminator (T), right homologous arm (HA) of YPRCdelta15, and plasmid backbone. Then, the plasmid pYR021 was obtained by recombinant construction in *Saccharomyces cerevisiae* using the same yeast assembly method as aforementioned, and sequentially ligating the above fragments. The plasmid construction schematic diagram of plasmid pYR021 is shown in FIG. 9 as pYR021, which also includes the Not I restriction site, and the rest part derives from the pRS426 plasmid backbone.

Primer sequences are shown in Table 6 below.

**Table 6**

| Primer | Sequence (5'-3') |
|---|---|
| 021-1F | ctaaagggaacaaaagcgcggccgcggcaatttggtacaaaaatcacg (SEQ ID NO.63) |
| 021-1R | ctatattatatatatagtaatgtcgtttttgcgaaaccctatgctc (SEQ ID NO.64) |
| 021-2F | gagcatagggtttcgcaaaaacgacattactatatatataatatag (SEQ ID NO.65) |
| 021-2R | gctgaatgggcagttcgaatacctgatgcggtattttctcc (SEQ ID NO.66) |
| 021-3F | ggagaaaataccgcatcaggtattcgaactgcccattcagc (SEQ ID NO.67) |
| 021-3R | gtaagaatttttgaaaattcaatataagtctgaagaatgaatgatttgatgat (SEQ ID NO.68) |
| 021-4F | aatcattcattcttcagacttatattgaattttcaaaaattcttactttttttttg (SEQ ID NO.69) |
| 021-4R | aacacaattgttgatgatcattatagttttttctccttgacg (SEQ ID NO.70) |
| 021-5F | gtcaaggagaaaaaactataatgatcatcaacaattgtgttacac (SEQ ID NO. 71) |
| 021-5R | cgatttcaattcaattcaatttaaaacatggagtttatatattccttgatg (SEQ ID NO.72) |
| 021-6F | tatataaactccatgttttaaattgaattgaattgaaatcg (SEQ ID NO.73) |
| 021-6R | gctcatcccgaccttccattaacgaacgcagaattttcgag (SEQ ID NO.74) |
| 021-7F | ctcgaaaattctgcgttcgttaatggaaggtcgggatgagc (SEQ ID NO.75) |
| 021-7R | agacggtcacagcttgtctgtgcggccgcgcttctaataaaccgatgaacgc (SEQ ID NO.76) |
| 021-8F | gcgttcatcggtttattagaagcgcggccgcacagacaagctgtgaccgtct (SEQ ID NO.77) |
| 021-8R | ttttgtaccaaattgccgcggccgcgcttttgttccctttagtgagg (SEQ ID NO.78) |

### 4.2 Construction of gene editing plasmids and knockout cassettes

Using plasmid pKIURA100 (see literature Zhang, Yueping et al."A gRNA-tRNA array for CRISPR-Cas9 based rapid multiplexed genome editing in Saccharomyces cerevisiae." Nature communications vol. 10,1 1053. 5 Mar. 2019, doi:10.1038/s41467-019-09005-3) as the template, amplify with primers 3951-F/R to obtain fragments. Goldengate method (Zhang, Yueping et al. "A gRNA-tRNA array for CRISPR-Cas9 based rapid multiplexed genome editing in Saccharomyces cerevisiae." Nature communications vol. 10,1 1053. 5 Mar. 2019, doi:10.1038/s41467-019-09005-3) was then used to assemble the above fragments and pCas to construct plasmid pYH395, and the plasmid construction schematic diagram is shown in FIG.10A.

Primer sequences are shown in Table 7 below.

**Table 7**

| Primer | Sequence (5'-3') |
|---|---|
| 3951-F | aaaggtctcagatcttttccactgcactttgcatgttttagagctagaaatagcaagtt (SEQ ID NO.79) |
| 3951-R | aaaggtctcaaaactctagactttttcgatgatgtagtttct (SEQ ID NO.80) |

Using the whole genome of *Saccharomyces cerevisiae* CEN.PK2-1D as the template, amplify with primers ERG9-1F/R and ERG9-2F/R to obtain two fragments, and then overlap extension PCR was performed with the obtained two fragments as the template to obtain ERG9 knockout cassette. A schematic diagram of the knockout element construction of the upstream activation *cis* element (-220 to-175) of the ERG9 promoter is shown in FIG. 10B, wherein the two HAs represent the homology arm to the left of the sequence (-220 to-175) and the homology arm to the right of the sequence (-220 to-175), respectively.

Primer sequences are shown in Table 8 below.

**Table 8**

| Primer | Sequence (5'-3') |
|---|---|
| ERG9-1F | atagaagacgaacattgtacgatac (SEQ ID NO.81) |
| ERG9-1R | acccaaaaccgataacgccttccgataagtcggtattgttgttgaagatg (SEQ ID NO.82) |
| ERG9-2F | tcaacaacaataccgacttatcggaaggcgttatcggttttgg (SEQ ID NO.83) |
| ERG9-2R | aatttctcgtggaagtgacg (SEQ ID NO.84) |

Using the whole genome of *Saccharomyces cerevisiae* CEN.PK2-1D, pRS426 plasmid and the whole genome of *Saccharomyces cerevisiae* CEN.PK2-1D as templates, amplify with primers 5211-F/R, 5212-F/R and 5213-F/R to obtain three fragments, and then overlap extension PCR was performed by using primers 5211-F and 5213-R with the obtained three fragments as templates to obtain pZY521 knockout cassette. The schematic diagram of the construction of pZY521 knockout cassette is shown in FIG. 11, in which the sequences are sequentially GAL80 left homology arm (HA), URA3 screening marker (promoter P_{URA3}, *URA3* gene, terminator T) and GAL80 right homology arm (HA).

Primer sequences are shown in Table 9 below.

**Table 9**

| Primer | Sequence (5'-3') |
|---|---|
| 5211_F | caatggtctaggtagtggcattcg (SEQ ID NO.85) |
| 5211-R | cgactcactatagggcgaattgggtacgacgggagtggaaagaacgg (SEQ ID NO.86) |
| 5212-F | tcccgttctttccactcccgtcgtacccaattcgccctatagtgag (SEQ ID NO.87) |
| 5212-R | gccaagcacagggcaagatgctttcacagcttgtctgtaagcgga (SEQ ID NO.88) |
| 5213-F | gcatccgcttacagacaagctgtgaaagcatcttgccctgtgctt (SEQ ID NO.89) |
| 5213-R | gattccatgctaccttccatggttg (SEQ ID NO.90) |

### 4.3 Strain construction

1) Plasmid pYR013 was digested with MssI (see the instruction manual of MssI enzyme for conditions of enzyme digestion system) to obtain linearized fragments, which were integrated into strain JCR27 by lithium acetate method to obtain strain CCJ-2.
2) Plasmid pYR020 was linearized with MssI enzyme and integrated into strain CCJ-2 in the same way as in step 1) to obtain strain CCJ-3.
3) Plasmid pYR021 was linearized with NotI enzyme and integrated into strain CCJ-3 in the same way as in step 1) to obtain strain CCJ-4.
4) Plasmid pYH395 and ERG9 knockout cassette were co-transferred into CCJ-4 by lithium acetate method, and the correctness of the knockout for the colonies were verified by PCR, before incubated in YPD liquid medium (20 g/L tryptone, 10g/L yeast extract,20 g/L glucose), with shaking at 220 rpm, at 30 °C for 8 hours, and then washed with water. After being streaked on 5-FOA plate (Xiaowei Li. Engineering Acetyl-CoA Pathway to Construct Efficient Biosynthesis Platform of Saccharomyces Cerevisiae [D]. Wuhan University, 2015.) and cultured in incubator at 30°C for 3 days, the strains were picked from the plate. The correct strain was named CCJ-5 after the correctness of the knockout for the colonies being verified by PCR.

This step is used to knock out the upstream activation *cis* element (-220 to -175) of ERG9 promoter, squalene synthase encoded by ERG9 synthesizes squalene by using FPP as the substrate, which is a competitive pathway of nerolidol synthesis pathway, and the knocking out of the upstream activation *cis* element of ERG9 promoter can downregulate the squalene synthesis pathway, reduce the competition of squalene synthesis pathway to consume the substrate required for nerolidol synthesis, and further improve the yield of nerolidol.

5) pZY521 knockout cassette was transferred into CCJ-5 by lithium acetate method to obtain CCJ-6. The step has the effect of knocking out transcription inhibitor GAL80, so that the target gene in the transferred bacteria can be expressed autonomously without an inducer, which is beneficial to reducing the cost of fermentation and further improving the yield of nerolidol.

### 4.4 Shake flask fermentation of the strain

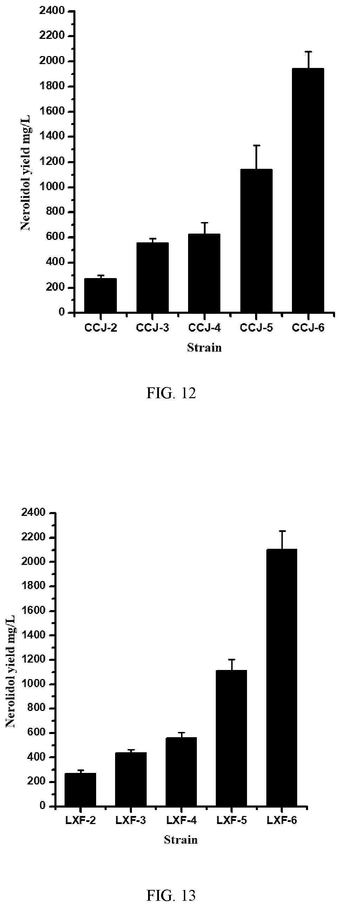

The strains CCJ-2, CCJ-3, CCJ-4, CCJ-5 and CCJ-6 were seeded respectively into YPD liquid medium and incubated under 30°C, 200 rpm with shaking overnight; transferred with an initial OD600=0.1 into 45 ml of YPDHG liquid medium (CCJ-2, CCJ-3, CCJ-4, and CCJ-5) or YPD liquid medium (CCJ-6) on the next day, followed by incubation for 72 hours under 30°C, 200 rpm shaking, with additional 5 ml isopropyl myristate. The oil layer was collected and the product was detected using GC-MS, under the same conditions as in 1.2.4 of Example 1. Nerolidol yield data for different strains are shown in FIG. 12. The results showed that the yield of nerolidol increased gradually with the increase of the copy number of the subject gene CCJ_TPS23, 271 mg/L for CCJ-2, 557 mg/L for CCJ-3, and 627 mg/L for CCJ-3. After downregulating of the squalene synthesis pathway (CCJ-5), nerolidol yield further increased significantly and reached 1140mg/L. Further knockout of galactose-induced transcription inhibitor GAL80 (CJ-6) leads to nerolidol yield further increased significantly and reached 1942 mg/L.

### Example 5 Construction of a High-yield Strain Comprising Nerolidol Synthetase ACH_TPS07 Derived from Astilbe chinensis

### 5.1 Construction of high yield plasmid

Using the whole genome of *Saccharomyces cerevisiae* CEN.PK2-1D, plasmid pRS423, the whole genome of *Saccharomyces cerevisiae* CEN.PK2-1D, the whole genome of *Saccharomyces cerevisiae* S288C, the whole genome of *Saccharomyces cerevisiae* CEN.PK2-1D, *Astilbe chinensis* cDNA, the whole genome of CEN.PK2-1D, the whole genome of CEN.PK2-1D, and pRS426 plasmid as templates, amplify with primers 020-1F/R, 020-2F/R, 020-3F/R, 020-4F/R, 020- 5F/017- 5R, 017-6F/R, 017-7F/020-7R, 020-8F/R and 020-9F/R, respectively, to obtain the fragments which are left homologous arm (HA) of Ura3, histidine screening marker (HIS3), CYC1 terminator (T), tHMG1 gene, GAL1-GAL10 promoters (P_{GAL10} and P_{GAL1}), ACH_TPS07, PGK1 terminator (T), right homologous arm (HA) of Ura3, and plasmid backbone. Then, the plasmid pYR017was obtained by recombinant construction in *Saccharomyces cerevisiae* using the same yeast assembly method as in Example 1, and sequentially ligating the above fragments. The plasmid construction schematic diagram of plasmid pYR017 is shown in FIG. 9 as pYR017, which also includes the MssI restriction site, and the rest part derives from the pRS426 plasmid backbone.

Primer sequences are shown in Table 10 below.

**Table 10**

| Primer | Sequence (5'-3') |
|---|---|
| 017-5R | aggaggaaggagggggtgccattatagttttttctccttgacgttaaagt (SEQ ID NO.91) |
| 017-6F | gtcaaggagaaaaaactataatggcaccccctccttcc (SEQ ID NO.92) |
| 017-6R | cgatttcaattcaattcaatttaaaacaaacattgtatatgctcctcaag (SEQ ID NO.93) |
| 0 17-7F | catatacaatgtttgttttaaattgaattgaattgaaatcgatagatcaat (SEQ ID NO.94) |

Using the whole genome of *Saccharomyces cerevisiae* CEN.PK2-1D, plasmid pRS424, the whole genome of *Saccharomyces cerevisiae* S288C, the whole genome of *Saccharomyces cerevisiae* CEN.PK2-1D, *Astilbe chinensis* cDNA, the whole genome of *Saccharomyces cerevisiae* CEN.PK2-1D, the whole genome of *Saccharomyces cerevisiae* CEN.PK2-1D, and pRS426 plasmid as templates, amplify with primers 021-1F/R, 021-2F/R, 021-3F/R, 021-4F/018-4R, 018-5F/R, 018-6F/021-6R, 021-7F/R and 021-8F/R, respectively, to obtain the fragments which are left homologous arm (HA) of YPRCdelta15, tryptophan selection marker (TRP1), GPM1 terminator (T), GAL1-GAL10 promoter (P_{CAL10} and P_{GAL1}), ACH_TPS07, PGK1 terminator (T), right homologous arm (HA) of YPRCdelta15, and plasmid backbone. Then, the plasmid pYR018was obtained by recombinant construction in *Saccharomyces cerevisiae* using the same yeast assembly method as aforementioned, and sequentially ligating the above fragments. The plasmid construction schematic diagram of plasmid pYR018 is shown in FIG. 9 as pYR018, which also includes the Not I restriction site, and the rest part derives from the pRS426 plasmid backbone.

Primer sequences are shown in Table 11 below.

**Table 11**

| Primer | Sequence (5'-3') |
|---|---|
| 018-4R | ggaggaaggagggggtgccattatagttttttctccttgacg (SEQ ID NO.95) |
| 018-5F | gtcaaggagaaaaaactataatggcaccccctccttcc (SEQ ID NO.96) |
| 018-5R | cgatttcaattcaattcaatttaaaacaaacattgtatatgctcctc (SEQ ID NO.97) |
| 018-6F | catatacaatgtttgttttaaattgaattgaattgaaatcg (SEQ ID NO.98) |

### 5.2 Strain construction

Plasmid pYR007 was linearized with MssI and integrated into strain JCR27 to obtain strain LXF-2, plasmid pYR017 was linearized with MssI and integrated into strain LXF-2 to obtain strain LXF-3, plasmid pYR018 was linearized with NotI and integrated into strain LXF-3 to obtain strain LXF-4, using the same method as in Example 4. Plasmid pYH395 and ERG9 knockout cassette were co-transferred into strain LXF-4, and the correctness of the knockout for the colonies were verified by PCR, before incubated in YPD liquid medium, and then washed with water. After being streaked on 5-FOA plate, the strains were picked from the plate. The correct strain was named LXF-5 after the correctness of the knockout for the colonies being verified by PCR; pZY521 knockout cassette was transferred into strain LXF-5 to obtain strain LXF-6.

### 5.3 Shake flask fermentation of the strain

The strains LXF-2, LXF-3, LXF-4, LXF-5, and LXF-6 were seeded respectively into YPD liquid medium and incubated under 30°C, 200 rpm with shaking overnight; transferred with an initial OD600=0.1 into 45 ml of YPDHG liquid medium ((LXF-2, LXF-3, LXF-4, and LXF-5) or YPD liquid medium (LXF-6) on the next day, followed by incubation for 72 hours under 30°C, 200 rpm shaking, with additional 5 ml isopropyl myristate. The oil layer was collected and the product was detected using GC-MS, under the same conditions as in 1.2.4 of Example 1. The yield data are shown in FIG. 13. Nerolidol yield data for different strains are shown in FIG. 13. The results showed that the yield of nerolidol increased gradually with the increase of the copy number of the subject gene ACH_TPS07, about 269 mg/L for LXF-2, about 436 mg/L for LXF-3, and about 556 mg/L for LXF-4. After downregulating of the squalene synthesis pathway (LXF-5), nerolidol yield further increased significantly and reached 1112mg/L. Further knockout of transcription inhibitor GAL80 (LXF-6) leads to nerolidol yield further increased significantly and reached 2105 mg/L.

### Example 6 Fermentor Fermentation of Nerolidol High-yield Strain

According to the fermentor medium and fermentation method described in literature (Ye Z, Huang Y, Shi B, et al. Coupling cell growth and biochemical pathway induction in Saccharomyces cerevisiae for production of (+)-valencene and its chemical conversion to (+)-nootkatone [published online ahead of print, 2022 Mar 13]. Metab Eng. 2022;72:107-115. doi:10.1016/j.ymben. 2022.03.005), the constructed strain LXF-6 was subjected to fed-batch fermentation, and a covering agent was added in the fermentation process to achieve *in situ* extraction, and the covering agent was isopropyl myristate. The fermentation process was controlled so that the dissolved oxygen is more than 20%, pH is 5, glucose concentration is 1-2g/L, and ethanol concentration is less than 5g/L. Eventually, the yield of nerolidol reached 55.7 g/L in 1 L fermentor, which is the highest level reported so far.

## Claims

1. A nerolidol synthase comprising domains of Pfam Nos. PF01397 and PF03936, and having activity of nerolidol synthase.

2. The nerolidol synthase according to claim 1, which is derived from *Tanacetum cinerariifolium, Astilbe chinensis* or *Artemisia argyi.*

3. The nerolidol synthase according to claim 1, having an amino acid sequence which has at least 90%, 91%, 92%, 93%, 94%, 95%, 96%, 97%, 98%, 99% or 100% sequence identity to the amino acid sequence as shown in SEQ ID NO. 1, SEQ ID NO. 2, SEQ ID NO. 3, SEQ ID NO. 4, SEQ ID NO. 11 or SEQ ID NO. 12.

4. A polynucleotide molecule comprising at least one of the nucleotide sequences encoding the nerolidol synthase according to any one of claims 1-3 or the complementary sequence thereof.

5. The polynucleotide molecule according to claim 4, comprising a nucleotide sequence which has at least 90%, 91%, 92%, 93%, 94%, 95%, 96%, 97%, 98%, 99% or 100% sequence identity to the nucleotide sequence as shown in SEQ ID NO.5, SEQ ID NO.6, SEQ ID NO.7, SEQ ID NO.8, SEQ ID NO.9 or SEQ ID NO.10.

6. A nucleic acid construct comprising at least one of the polynucleotide molecules according to claim 4 or 5.

7. The nucleic acid construct according to claim 6, further comprising a nucleotide sequence which encodes at least one selected from acetoacetyl coenzyme A thiolase, hydroxy-methylglutaryl coenzyme A synthase, hydroxy-methylglutaryl coenzyme A reductase, mevalonate kinase, mevalonate-5-phosphate kinase, mevalonate pyrophosphate decarboxylase, isoprene pyrophosphate isomerase and farnesyl pyrophosphate synthase.

8. The nucleic acid construct according to claim 6 or 7, wherein the nucleotide sequence is located between two insertion elements, wherein the insertion elements are used for integrating the nucleotide sequence into the genome of a host cell.

9. The nucleic acid construct according to claim 6, which is a plasmid vector; preferably, the plasmid vector is a eukaryotic expression vector.

10. The nucleic acid construct according to claim 9, comprising a pRS426 plasmid backbone.

11. The nucleic acid construct according to claim 10, which is at least one plasmid vector selected from pYR006, pYR007, pYR010, pYR013, pAra-TPS27, pAra-TPS28, pYR017, pYR018, pYR020, and pYR021, wherein the construction schematic diagram of the plasmid vectors is as shown in FIG. 2 or FIG. 9.

12. A recombinant strain comprising the polynucleotide molecule according to claim 4 or 5, or the nucleic acid construct according to any one of claims 6-11.

13. The recombinant strain according to claim 12, wherein the polynucleotide molecule is integrated into the genome of a host cell; preferably, the host cell is a eukaryotic cell, more preferably is *Saccharomyces cerevisiae.*

14. The recombinant strain according to claim 13, wherein the copy number of the polynucleotide molecule in the genome of the recombinant strain is 1-3.

15. The recombinant strain according to claim 12, which is capable of endogenously and/or exogenously expressing at least one selected from acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase.

16. The recombinant strain according to claim 15, wherein the copy numbers of nucleotide sequences encoding acetoacetyl coenzyme A thiolase, hydroxy-methylglutaryl-CoA synthase, hydroxy-methylglutaryl-CoA reductase, mevalonate kinase, mevalonate-5-phosphate kinase, mevalonate pyrophosphate decarboxylase, isoprene pyrophosphate isomerase and farnesyl pyrophosphate synthase in the genome of the recombinant strain are 2, 2, 4, 2, 2, 2, 2, and 2, respectively.

17. The recombinant strain according to claim 12, further comprising downregulation or knockout of at least one of the genes encoding FPP hydrolase DPP1, FPP hydrolase LPP1, citrate synthase, malate synthase, or squalene synthase.

18. Use of the nerolidol synthase according to any one of claims 1-3, the polynucleotide molecule according to claim 4 or 5, the nucleic acid construct according to any one of claims 6-11 or the recombinant strain according to any one of claims 12-17 in the production of nerolidol.

19. A method for preparing nerolidol, comprising biosynthesizing nerolidol by using the recombinant strain according to any one of claims 12-17.
